(19) Europäisches Patentamt
European Patent Office
Office européen des brevets

(11) EP 4 582 078 A1

(12) EUROPEAN PATENT APPLICATION

(43) Date of publication:
**09.07.2025 Bulletin 2025/28**

(21) Application number: **24205448.4**

(22) Date of filing: **09.10.2024**

(51) International Patent Classification (IPC):
***A61K 8/55*** *(2006.01)* ***A61K 8/63*** *(2006.01)*
***A61K 9/00*** *(2006.01)* ***A61K 31/575*** *(2006.01)*
***A61K 31/685*** *(2006.01)* ***A61P 43/00*** *(2006.01)*
***A61Q 19/06*** *(2006.01)*

(52) Cooperative Patent Classification (CPC):
(C-Sets available)
**A61K 31/685; A61K 8/553; A61K 8/63;**
**A61K 31/575; A61P 43/00; A61Q 19/06;**
A61K 9/0019; A61K 2800/91 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.01.2024 KR 20240001301**

(71) Applicant: **AMI Pharm Co., Ltd.**
**Seongnam-si, Gyeonggi-do 13215 (KR)**

(72) Inventor: **LEE, Ki-Taek**
**06006 Seoul (KR)**

(74) Representative: **Hoefer & Partner Patentanwälte mbB**
**Pilgersheimer Straße 20**
**81543 München (DE)**

(54) **COMPOSITION FOR LOCALIZED FAT REDUCTION WITHOUT PAIN, EDEMA AND SIDE EFFECTS AND METHOD OF ADMINISTRATION THEREOF**

(57) The present invention relates to a composition useful for reducing localized fat using phosphatidylcholine for a subject with localized fat deposits without pain, edema or side effects, and a method of administration thereof, and, more particularly, to a composition for localized fat reduction with reduced pain and side effects, comprising (i) phosphatidyl choline; and (ii) at least one selected from the group consisting of glycocholic acid (GCA), taurocholic acid (TCA) and salts thereof, wherein (i) and (ii) have a molar ratio of (ii)/(i) of 0.7 to 3.0, and wherein the composition is administered to the affected area of the subject at intervals of less than 4 weeks at a concentration of 25 to 50 mg/mL. The composition of the present invention provides a safe and stable formulation and is highly effective in reducing fat cells without various side effects, and may be administered to the affected area at shorter intervals to maximize the effects.

[FIG. 3]

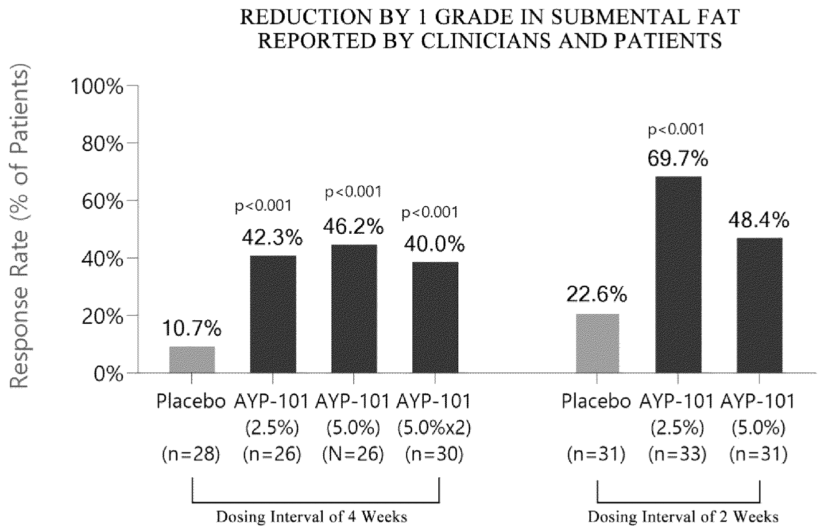


EP 4 582 078 A1

(52) Cooperative Patent Classification (CPC): (Cont.)

C-Sets
**A61K 31/575, A61K 2300/00;**
**A61K 31/685, A61K 2300/00**

## Description

### TECHNICAL FIELD

The present invention relates to a composition useful for reducing localized fat using phosphatidylcholine for a subject with localized fat deposits without pain, edema or side effects, and a method of administration thereof, and, more particularly, to a composition for localized fat reduction with reduced pain and side effects, comprising (i) phosphatidyl choline; and (ii) at least one selected from the group consisting of glycocholic acid (GCA), taurocholic acid (TCA) and salts thereof, wherein (i) and (ii) have a molar ratio of (ii)/(i) of 0.7 to 3.0, and wherein the composition is administered to the affected area of the subject at intervals of less than 4 weeks at a concentration of 25 to 50 mg/mL.

### BACKGROUND ART

Localized fat deposits are of particular concern to many people. Unwanted fat deposits on the face or other parts of the body may make a person look less attractive and older. They may occur due to aging, lifestyle or genetic predisposition. To overcome them, efforts are made to reduce them through exercise and diet, but the fat reduction effect is limited.

Methods for reducing locally fat deposits include cosmetic surgical procedures and cosmetic minimally-invasive procedures. Cosmetic surgical procedures take a long time to heal and may significantly delay healing for certain people such as smokers and diabetics, and involve potential complications and risks such as potential complications and risks such as fatal side effects, risks of general anesthesia, excessive bleeding, damage to internal organs, bacterial infection, scarring, bruising, edema and pain. Since the safety and efficacy of cosmetic minimally-invasive procedures have not been secured due to the absence of large-scale clinical trials such that the procedures contain potential risks, there is a need to develop new clinically beneficial medicines for localized fat reduction.

Injectable drugs for localized fat reduction are drugs that induce fat cell reduction by injecting drugs into the subcutaneous fat layer, and a representative example is PPC (Polyene Phosphatidylcholine) injection. The previously known PPC injection is an injection that uses deoxycholic acid (DCA) as a solubilizer, and its fat-reducing pharmacologically active ingredient is known as DCA. This DCA alone or PPC injection solubilized with DCA non-selectively dissolves not only adipocytes (3T3L1 adipocytes) but also normal fibroblasts, vascular endothelial cells and skeletal muscle cells and, thus, is a cell-lytic injection, rather than a fat-dissolving injection (Non-patent Document 1).

Accordingly, the present inventor confirmed that PPC-only composition without DCA induced apoptosis, rather than necrosis, in adipocytes, reducing only adipocytes and having no effect on reducing fibroblasts (non- Patent Document 2), and, through additional research based on thereon, confirmed that a composition prepared by adding taurocholic acid (TCA), especially glycocholic acid (GCA), to phosphatidylcholine (PPC) at a specific mixing ratio had excellent safety and formulation stability and caused fat reduction without pain, edema and side effects, and completed the invention (Patent Document 1).

Meanwhile, treatment with DCA alone or PPC injection solubilized with DCA administered multiple times can cause local adverse events such as inflammation and resulting edema, pain, erythema, and hematoma that takes 4 weeks to resolve. Therefore, it is known that the administration of injections with an interval of at least 4 weeks or more between each administration is a general treatment method (Non-Patent Document 3, Non-Patent Document 4, Non-Patent Document 5, Non-Patent Document 3 and Patent Document 6).

### PRIOR ART CITATIONS

### PATENT CITATIONS

Korean Patent No. 1887586

### NON-PATENT CITATIONS

A.Gupta, Action and comparative efficacy of phosphatidylcholine formulation and isolated sodium deoxycholate for different cell type, Aest Plast Sur, 33: 346-352, 2009

Dong-Seok Kim, Phosphatidylcholine induces apoptosis of 3T3-L1 adipocytes, Journal of biomedical science, 18: 91, 1-7, 2011

Shamban AT. Noninvasive Submental Fat Compartment Treatment. Plast Reconstr Surg Glob Open. 2016 Dec 14; 4 (12 Suppl Anatomy and Safety in Cosmetic Medicine: Cosmetic Bootcamp): e1155.

Thomas MK, D'Silva JA, Borole AJ. Injection Lipolysis: A Systematic Review of Literature and Our Experience with a Combination of Phosphatidylcholine and Deoxycholate over a Period of 14 Years in 1269 Patients of Indian and

South East Asian Origin. J Cutan Aesthet Surg. 2018 Oct-Dec; 11(4): 222-228.

KYBELLA (deoxycholic acid) injection. Allergan. 2018; package insert.

ADAM M. ROTUNDA, MD,_ STEVEN R. WEISS, MD,AND LARRY S. RIVKIN, MD Randomized Double-Blind Clinical Trial of Subcutaneously Injected Deoxycholate Versus a Phosphatidylcholine-Deoxycholate Combination for the Reduction of Submental Fat, Dermatol Surg 2009; 35: 792-803.

## CONTENTS OF THE INVENTION

### TECHNICAL PROBLEMS

While conducting additional research on the composition prepared by adding taurocholic acid (TCA), especially glycocholic acid (GCA), to phosphatidylcholine (PPC) at a specific mixing ratio, the present inventor confirmed that the effect of the injectable composition was maximized by administering it at shorter intervals, rather than interval of 4 weeks or more as conventionally known in the art, and improved patient convenience, and completed the present invention.

Therefore, the objective of the present disclosure is to provide a composition for localized fat reduction, comprising (i) phosphatidyl choline; and (ii) at least one selected from the group consisting of glycocholic acid (GCA), taurocholic acid (TCA) and salts thereof, wherein (i) and (ii) have a molar ratio of (ii)/(i) of 0.7 to 3.0, and wherein the composition is administered to the affected area of a subject at intervals of less than 4 weeks at a concentration of 25 to 50 mg/mL.

### MEANS OF SOLVING PROBLEM

In order to achieve the above-described objective, there is provided a composition for localized fat reduction, comprising (i) phosphatidyl choline; and (ii) at least one selected from the group consisting of glycocholic acid (GCA), taurocholic acid (TCA) and salts thereof, wherein (i) and (ii) have a molar ratio of (ii)/(i) of 0.7 to 3.0, and wherein the composition is administered to the affected area of a subject once every two weeks at a concentration of 25 to 50 mg/mL.

Hereinafter, the present invention will be described in detail.

Unless otherwise defined, all technical and scientific terms used herein have the same meaning as commonly understood by a person of ordinary skill in the technical field to which the present invention belongs. Although any methods and materials similar or equivalent to those described herein may be used in the practice or examination of the present invention, the preferred methods and materials are described.

The present inventor administered the test drug (AYP-101) according to the present invention, which is phosphatidyl choline (PPC) solubilized with glycocholic acid (GCA), by classifying the subjects into those administered at 4-week intervals, i.e., Test Group 1 (AYP-101 25 mg/mL, 0.2 mL/Point), Test Group 2 (AYP-101 50 mg/mL, 0.2 mL/Point), Test Group 3 (AYP-101 50 mg/mL, 0.4 mL/Point) and control group (AYP-101 0 mg/mL, 0.2 mL/Point); and those administered at 2-week intervals, i.e., Test Group 1 (AYP-101 25 mg/mL), Test Group 2 (AYP-101 50 mg/mL) and control group (placebo of AYP-101), and confirmed the effects.

As a result, in the case of the test groups administered at 4-week intervals, at 12 weeks after the final administration, the proportion of the subjects whose main endpoints, the Clinician-Reported Submental Fat Rating Scale (CR-SMFRS) and the Patient-Reported Submental Fat Rating Scale (PR-SMFRS), were reduced by 1 grade or more, as compared to the baseline value, was 42.31% (11/26 people) in Test Group 1, 46.15% (12/26 people) in Test Group 2, 40.00% and (12/30 people) in Test Group 3. As such, it was confirmed that the proportion of the subjects in all test groups were superior to that in the control group (10.71% (3/28 people)). In the case of the test groups administered at 2-week intervals, at 4 weeks after the final administration, the proportion of the subjects whose main endpoints, the Estimator-Reported Submental Fat Rating Scale (ER-SMFRS) and the Subject-Reported Submental Fat Rating Scale (SR-SMFRS), were reduced by 1 grade or more, as compared to the baseline value, was 69.70% (23/33 people) in Test Group 1, 48.39% (15/31 people) in Test Group 2, and 22.58% (7/31 people) in the control group. That is, the effect of reducing submental fat was greater in both Test Groups 1 and 2, as compared to that in the control group. Thus, the effect of the administration of the test drug at the dose of 25 mg/mL every 2 weeks was more significant, as compared to that of the administration every 4 weeks.

Therefore, the present invention provides a composition for localized fat reduction, comprising (i) phosphatidyl choline; and (ii) at least one selected from the group consisting of glycocholic acid (GCA), taurocholic acid (TCA) and salts thereof, wherein (i) and (ii) have a molar ratio of (ii)/(i) of 0.7 to 3.0, and wherein the composition is administered to the affected area of a subject at intervals of less than 4 weeks at a concentration of 25 to 50 mg/mL.

For the detailed description of the composition according to the present invention, i.e., the composition according to the present invention, which is PPC solubilized with GCA, may refer to Korean Patent No. 1887586, which is a prior document, and the entire document is incorporated by reference herein.

The composition according to the present invention is not particularly limited in its formulation as long as it is used

for the purpose of fat reduction (particularly localized fat reduction), but comprises, for example, injections such as patches and subcutaneous depots, and may preferably be a composition of an injectable preparation (an injection). That is, the present invention relates to a composition that may be directly injected into the treated area of a patient in need of fat reduction without intervention of surgery.

The injectable composition of the present invention may be administered by a method suitable for the patient or subject in terms of the severity of the disease, and the patient's age, gender, and other conditions, but the present invention is not limited thereto. As the routes of administration, the composition may be directly administered to the subcutaneous fat layer (tissue), or administered by subcutaneous injection or intradermal injection, but the present invention is not limited thereto.

The subcutaneous or intradermal injection is preferably administered multiple times to the same affected area, and between each administration, there may be an interval of 1 week or more and less than 4 weeks, preferably 1 week or more and 3 weeks or less, more preferably 1 week or more and 2 weeks or less, and most preferably 2 weeks, but the present invention is not limited thereto. When the subcutaneous or intradermal injection is administered multiple times, the total number of administrations can be 2, 3, 4, 5, 6, 7, 8, 9, or 10 times, and preferably 4 to 8 times, but the present invention is not limited thereto. That is, according to the compositions and administration methods as conventionally known in the art, the total period required for treatment may extend from a minimum of 4 weeks (administered twice) to a maximum of 40 weeks (administered 10 times), and typically 16 to 32 weeks (administered 4 to 8 times) are required for treatment, whereas according to the compositions and the administration methods of the present invention, a minimum of 2 weeks (administered twice) to a maximum of 20 weeks (administered 10 times) is sufficient for the total period, and typically, treatment can be done in 8 to 16 weeks (administered 4 to 8 times). Meanwhile, when administered once, the injectable composition according to the present invention is preferably administered at a concentration of 25 to 50 mg/mL.

When administered once, the injectable composition according to the present invention may be administered by setting several target sites (i.e., injected points or points) at regular intervals to the affected area. The terms 'target site', 'injected point' or 'point' refer to the point at which an injection is made to the affected area, and the terms are used interchangeably in this specification. At this time, the intervals of the target sites may be grid intervals of 0.5 to 2.0 cm, preferably 0.7 to 1.3 cm, and most preferably 1.0 cm, and the number of target sites (i.e., injected points or points) may be appropriately determined by considering various conditions. The amount of administration may be 0.1 to 0.5 mL, preferably 0.2 to 0.4 mL, and most preferably 0.2 mL, for each target site (i.e., injected point or points), and considering all these conditions, the composition according to the present invention can be administered to the affected area at 2.5 mg to 25 mg, preferably 5 mg to 20 mg, and most preferably 5 mg to 10 mg of phosphatidylcholine per 1.0 $cm^2$ of target sites (i.e., injected points or points), but the present invention is not limited thereto.

The composition of the present invention may be administered at various levels (depths) under the skin, for example, comprising 0.1 to 4 inches, 0.5 to 3 inches, and 1 to 2 inches below the skin, but present invention is not limited thereto.

The injectable composition for fat reduction of the present invention may be applied to localized areas and to the reduction of neck wrinkles and fat and lipoma areas deposited on the areas which may be preferably abdomen, under the chin, forearms, thighs, waist, buttocks, under the eyes and bra line (brassiere line), but present invention is not limited thereto.

The injectable composition for localized fat reduction of the present invention is a pharmaceutical composition for the treatment of adipose tissue hyperproliferation or hyperaccumulation disorder (disease), and the type of the disorder is not particularly limited as long as it is known in the art to cause pathological overproliferation or overaccumulation of adipose tissue, but comprises, for examples, obesity (e.g., abdominal obesity), lower eyelid prolapse, lipoma, Dercum's disease, Madelung's neck, lipedema, piezogenic nodules, xanthelasma, lipodystrophy or fat accumulation associated with cellulite.

Injections are made by dissolving the main drug (PPC solubilized with GCA in the present invention) and, if necessary, other additives in water for injection, filtering the solution through a bacterial filter to treat same aseptically, filling same in a sterile state into vials, ampoules, or prefilled syringes and sealing same. When preparing an injection, water for injection other than water may be used to fill the remaining amount. The type of water for injection is not particularly limited as long as it is distilled water for injection or a buffer solution for injection made to dissolve solid injections or dilute water-soluble injections, but may comprise, for example, a phosphate buffer solution with a pH range of 3.5 to 7.5 and a sodium dihydrogen phosphate ($NaH_2PO_4$)-citric acid buffer solution. The phosphate used at this time may be in the form of a sodium salt or a potassium salt, or its anhydrous or hydrated form, and may be in the form of citric acid, or its anhydrous or hydrated form. In addition, examples of water for injection comprise glucose injection, xylitol injection, D-mannitol injection, fructose injection, saline solution, Dextran 40 injection, Dextran 70 injection, amino acid injection, Ringer's solution and lactic acid-Ringer's solution, but the invention is not limited thereto.

The following description relates to the composition according to the present invention (i.e., PPC solubilized with GCA) itself, and more detailed information is described in Korean Patent No. 1887586, a prior document.

As used herein, the term 'Phosphatidylcholine' refers to a phospholipid, a compound represented by the IUPAC

Name 1,2-diacyl-sn-glycero-3-phosphocholine, and is referred to herein as PPC.

As used herein, the term "bile acid" comprises steroid acids (and/or their carboxylic acid anions), and salts thereof, is found in the bile of animals (e.g., human beings). 'Deoxycholic acid' is a type of bile salt and refers to the compound represented by IUPAC Name (4R)-4-[(3R,SR,8R,9S,10S,12S,13R,14S,17R)-3,12-dihydroxy-10,13-dimethyl-2,3,4,5,6,7,8,9,11,12,14,15, 16,17-tetradecahydro-1H-cyclopenta[a]phenanthren-17-yl]pentanoic acid. It is referred to herein as DCA.

'Glycocholic acid' is a type of bile salt and refers to a compound denoted by IUPAC Name 2-[[(4R)-4-[(3R,5S,7R,8R,9S,10S,12S,13R,14S,17R)-3,7,12-trihydroxy-10,13-dimethyl-2,3,4, 5,6,7,8,9,11,12,14,15,16,17-tetradecahydro-1H-cyclopenta[a]phenanthren-17-yl]pentanoyl]amino] acetic acid. It is described herein as GCA.

'Taurocholic acid' is a type of bile salt and refers to the compound indicated by IUPAC Name 2-[[(4R)-4-[(3R,5S,7R,8R,9S,10S,12S,13R,14S,17R)-3,7,12-trihydroxy-10,13-dimethyl-2,3,4, 5,6,7,8,9,11,12,14,15,16,17-tetradecahydro-1H-cyclopenta[a]phenanthren-17-yl]pentanoyl]amino] ethanesulfonic acid. It is referred to herein as TCA.

In the present invention, phosphatidylcholine (PPC) is a phospholipid widely present in animals, plants, yeast and molds, is also called lecithin, polyenephosphatidylcholine and 3-sn-phosphatidylcholine, and has the same basic structure as [Formula 1] below. It is a mammalian membrane phospholipid, mainly contained in brain water, nerves, blood cells, and egg yolk. In plants, it is contained in soybeans, sunflower seeds, and wheat germ, but is rarely found in bacteria. Glycerol generally has a saturated fatty acid bound to position 1 and an unsaturated fatty acid bound to position 2, and most of the acyl groups are C12 to C22 (12 to 22 carbon atoms).

[Formula 1]

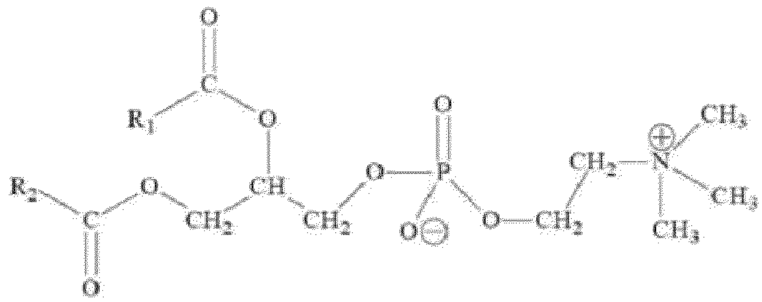

Phosphatidylcholine of the present invention has the same structure as [Formula 1] above, wherein $R^1$ may be a saturated or unsaturated fatty acid having 12 to 22 carbon atoms, and $R^2$ may be a saturated or unsaturated fatty acid having 12 to 22 carbon atoms. The saturated or unsaturated fatty acid may be in a straight chain or branched chain form, and the unsaturated fatty acid may comprise monounsaturated or poly (e.g., double, triple, or quadruple) unsaturated fatty acid. Phosphatidylcholine of the present invention may be a single compound or a mixture of several compounds with various carbon atoms in the R1 and R2 acyl groups. Preferably, the phosphatidylcholine of the present invention may have a molecular weight of 700 g/mol to 1000 g/mol, and more preferably, may have a molecular weight of 750 g/mol to 800 g/mol.

Phosphatidylcholine of the present invention may be extracted and used from various animals or plants, for example, any one selected from the group consisting of soybeans, sunflower seeds, wheat germ, and egg yolk. Alternatively, the phosphatidylcholine of the present invention may be purchased and used commercially, or may be manufactured by a chemical synthesis method known in the art.

Phosphatidylcholine of the present invention may preferably be isolated from soybean or egg yolk. The typical structure of phosphatidylcholine generally isolated from soybeans is shown in [Formula 2] below. In addition, the typical structure of phosphatidylcholine, which is generally derived from egg yolk, is shown in [Formula 3] below. The phosphatidylcholine used in the present invention may be a single compound consisting only of compounds of the following Formula 2 or Formula 3, or may be a mixture added with several compounds with various carbon atoms of the $R^1$ and $R^2$ acyl groups having the basic structure of Formula 1. The compound of Formula 2 or Formula 3 below may be contained in the mixture in an amount of substantially 50% by weight or more, more preferably 70% by weight or more, and most preferably 90% by weight or more.

[Formula 2]

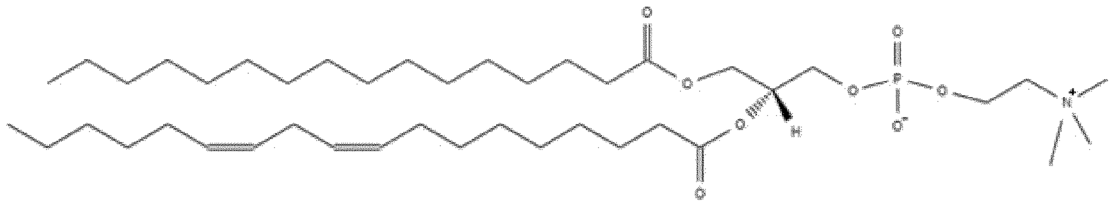

[Formula 3]

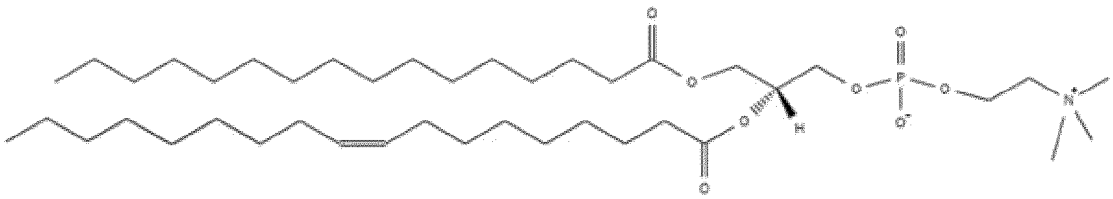

Most preferably, the phosphatidylcholine of the present invention may be extracted from soybeans, and may be a mixture containing a compound having the same structure as [Formula 2] in a ratio of 93.0% by weight or more.

In the injectable composition for localized fat reduction of the present invention, the phosphatidylcholine may be contained in an amount of 0.625 to 15.0% (w/v) based on the amount of the total composition, and preferably 1.25 to 12.5% (w/v), more preferably, 2.5 to 10.0 (w/v)% based on the amount of the total composition. Most preferably, the phosphatidylcholine may be contained in an amount of 2.5 to 7.5% (w/v), based on the amount of the total composition. If the concentration of phosphatidylcholine is less than 0.625% (w/v), it has no lipolysis effect. If the concentration exceeds 15% (w/v), it is inconvenient to administer multiple times to the subcutaneous fat layer due to its high viscosity, and excessive addition of a solubilizer is required such that moderate to severe inflammatory response may occur, resulting in severe side effects such as pain, edema and inflammation.

The composition for localized fat reduction of the present invention is characterized in that it comprises at least one selected from the group consisting of glycocholic acid, taurocholic acid, and salts thereof (hereinafter,(ii)), and phosphatidylcholine (PPC) (hereinafter, (i)) at a molar ratio of (ii)/(i) of 0.7 to 3.0. As such, the molar ratio of (ii)/(i) may be 0.7 to 3.0, more preferably, 0.7 to 2.60, and most preferably 0.7 to 1.73. If they are contained at a molar ratio (mol/mol) of less than 0.7, it is difficult to form stable micelles such that the formulation safety is reduced, and, thus, the lower limit of the molar ratio is preferably 0.70 or more, and more preferably 0.76. If the upper limit of the molar ratio is 3.04 or more, pain, edema and side effects due to mild or more inflammation, moderate or more edema, and more severe skin lesions appear significantly, and cell necrosis are accompanied, thereby limiting the unique function of PPC, rather than imparting a positive effect on the unique fat cell apoptosis and lipolysis of PPC. At a molar ratio of 3.0 or less, such side effects and pain are significantly reduced, and, especially at a molar ratio of 2.60 or less, edema, lesions and inflammation are confirmed to be absent or mild. Although edema may clinically appear mild, but it has substantially no pain or side effects. Thus, the molar ratio is adopted as a more preferable range in the present invention. Most preferably, when the molar ratio is 1.73 or less, clinical pain and edema due to edema, lesions and inflammation do not occur.

Specifically, the composition for localized fat reduction of the present invention is characterized in that it comprises 'glycocholic acid or a salt thereof in a specific mixing ratio in the composition. The glycocholic acid is a bile salt, has a molecular weight of about 465.63 g/mol, and may be described herein as GCA or GC. The glycocholic acid may be used in the form of a pharmaceutically acceptable salt. As used herein, 'pharmaceutically acceptable' refers to being physiologically acceptable and not normally causing allergic or similar reactions when administered to human beings, and, but not limited to, may be, for example, a sodium salt, a potassium salt, or an ammonium salt. Preferably, the glycocholate of the present invention may be sodium glycocholate (GCNa).

The glycocholic acid or salt thereof may be extracted from the intestines of animals according to a method known in the art, and may be purchased commercially, or manufactured by a chemical synthesis method known in the art.

More specifically, as the minimum mixing composition for producing a clear solution capable of being microfiltered using a mixed micelle preparation with 10 nm or less that may be stably and safely injected subcutaneously, the minimum molar ratio of GCA/PPC is 0.76 (PPC 5.0% + GCA 2.2%). If the molar ratio is less than the minimum molar ratio, precipitation occurs and the stability of the formulation is low. Therefore, the glycocholic acid or a salt thereof may preferably be contained in a molar ratio of GCA/PPC of 0.76 to 3.0 (GCA 2.2 to 8.65% (w/v) based on 5% PPC), and the specific range is as described above in relation to the molar ratio. When glycocholic acid is used in its salt form, the molar

ratio may be preferably calculated based on only the glycocholic acid portion of the glycocholic acid salt.

The composition for localized fat reduction of the present invention is characterized in that it comprises 'taurocholic acid or a salt thereof in a specific mixing ratio in the composition. The taurocholic acid is a bile salt, has a molecular weight of about 515.71 g/mol, and may be described herein as TCA. The taurocholic acid may be used in the form of a pharmaceutically acceptable salt. As used herein, 'pharmaceutically acceptable' refers to being physiologically acceptable and not normally causing allergic or similar reactions when administered to human beings, and may comprise for example, a sodium salt, a potassium salt, or an ammonium salt. Preferably, the taurocholate of the present invention may be sodium taurocholate (TCNa), but the present invention is not limited thereto.

The taurocholic acid or salt thereof may be extracted from the intestines of animals according to methods known in the art, purchased commercially or manufactured by chemical synthesis methods known in the art.

More specifically, as the minimum mixing composition for producing a clear solution capable of being microfiltered using a mixed micelle preparation with 10 nm or less that may be stably and safely injected subcutaneously, the minimum molar ratio of TCA/PPC is 0.78 (PPC 5% + TCA 2.5%). If the molar ratio is less than the minimum molar ratio, precipitation occurs and the stability of the formulation is low. Therefore, the taurocholic acid or a salt thereof may preferably be contained in a molar ratio of TCA/PPC of 0.78 to 3.0 (TCA 2.5 to 9.57% (w/v) based on 5% PPC), and the specific range is as described above in relation to the molar ratio. When taurocholic acid is used in its salt form, the molar ratio may be preferably calculated based on only the taurocholic acid portion of the glycocholic acid salt.

The GCA, TCA or salts thereof are contained in the injectable composition for fat reduction of the present invention at a specific dose (or mixing ratio or molar ratio) as described above, thereby providing excellent formulation stability and, together with PPC, induce highly efficient lipolysis and apoptosis to reduce pain and side effects such as hematoma, numbness, erythema, swelling, hardening, itching and nodules to the extent that they are substantially free, unlike existing solubilizers (especially, deoxycholate and a salt thereof) in PPC injectable compositions which cause necrosis of cells, involving pain and edema in the body and causing such side effects, and the GCA, TCA or salts thereof also show an excellent effect in fat reduction. (Pain and edema were reduced by more than 80%, and erythema, hematoma, hardening, itching and nodules, except for bruises caused by injection needles, are also reduced by more than 80%.) Accordingly, they also have a special advantage in that there is no need to necessarily add or use separate anti-inflammatory or/and analgesic ingredients in the composition for pain management.

Meanwhile, the composition of the present invention may further comprise at least one selected from the group consisting of a preservative; an isotonic agent; and a pH adjuster.

Specifically, the composition for localized fat reduction of the present invention may further comprise at least one substance selected from the group consisting of preferably 0.1 to 5% (w/v) of a preservative, 0.1 to 10% (w/v) of an isotonic agent and 0.01 to 2% (w/v) of a pH adjuster, based on the amount of the total composition.

The preservative may be selected from the group consisting of benzyl alcohol, lidocaine, procaine and chlorobutanol, and more preferably may be benzyl alcohol, but the present invention is not limited thereto. The benzyl alcohol is one of aromatic alcohols and is a colorless and transparent liquid. The concentration of benzyl alcohol contained in the injectable composition of the present invention may preferably be 0.1% (w/v) to 2% (w/v).

The isotonic agent serves to appropriately maintain (adjust) the osmotic pressure when the composition of the present invention comprising phosphatidylcholine is administered into the body, and also has the secondary effect of further stabilizing phosphatidylcholine in a solution. The isotonic agent may be a pharmaceutically acceptable sugar, salt, or any combination or mixture thereof, examples of which may be glucose as a sugar, and sodium chloride, calcium chloride, sodium sulfate, glycerin, propylene glycol or polyethylene glycol of 1000 or less as water-soluble inorganic salts, and may more preferably be sodium chloride. They may be used alone or in combination of two or more. The concentration of the isotonic agent is preferably 0.1% (w/v) to 5% (w/v), and may be adjusted to an appropriate content such that the solution formulation comprising all of the respective mixtures depending on the type and amount of ingredients contained in the composition of the present invention becomes an isotonic solution.

The pH adjuster of the present invention serves to adjust the pH of the injection and comprises both acidic and basic substances. The acidic substances comprise hydrochloric acid, acetic acid, adipic acid, ascorbic acid, sodium ascorbate, sodium etherate, malic acid, succinic acid, tartaric acid, fumaric acid and citric acid (citric acid), but the present invention is not limited thereto. The basic substances comprise inorganic bases (e.g. sodium hydroxide, potassium hydroxide, sodium carbonate, sodium bicarbonate, magnesium carbonate, calcium carbonate, magnesium oxide, ammonia, synthetic hydrotalcite), and organic bases (e.g. basic amino acids such as lysine, arginine and meglumine), but the present invention is not limited thereto. In the present invention, as the pH adjuster, an acidic substance and a basic substance may be contained alone in the composition, or two or more of each substance may be used in combination. More preferably, the pH adjuster of the present invention may be sodium hydroxide or/and hydrochloric acid. The amount of the pH adjuster to be added may vary depending on the type and amount of the components of the composition of the present invention, and may be preferably 0.01% (w/v) to 1.32% (w/v), more preferably 0.01% (w/v) to 1% (w/v). The composition of the present invention may preferably be provided in the range of pH 6.5 to pH 8.5, and the type and amount of the pH adjuster to be added may be changed by a person skilled in the art depending on the specific composition of the

solution.

As used herein, the term "side effects" refers to the side effects of a PPC preparation comprising a specific bile salt that is known or commercially available, especially a PPC injection solubilized with DCA, which is known as an injection for localized fat reduction (e.g., Lipostabil, Essential, Lipovine) or a DCA single agent (e.g. Kybella), and refers to an effect harmful to the human body other than the main effect of the drug expected to have a therapeutic effect (in the present invention, the target fat reduction effect). Specifically, the term refers to at least any one selected from the group consisting of a, b, and c, except for hematomas and bruises caused by injection needles, but the present invention is not limited thereto. The injectable composition for localized fat reduction of the present invention is characterized in that it reduces local adverse reactions, and reduces the above-mentioned side effects to the extent that they are substantially free. Edema, numbness (especially, numbness at the injected site), extensive swelling, erythema, hematoma, bruising, induration, paresthesia, nodules, itching, burning, difficulty swallowing, necrosis of other cells than adipocytes (e.g., muscle cells, fibroblasts and vascular endothelial cells).

As used herein, the term "pain and side effects are reduced" comprise all of the cases that pain and side effects are reduced, eliminated, mildly (partially eliminated), substantially absent (substantially eliminated), or completely absent (completely removed).

As used herein, the terms "patient," "subject" and "individual" are used interchangeably and refer to any animal that are amenable (in vitro or in situ) to the methods described herein, or its cells. In certain non-limiting embodiments, the patient, subject or individual is a human being.

As used herein, the term "composition" or "pharmaceutical composition" may encompass all mixtures of at least one compound or composition and other chemical components such as any additional carriers, stabilizers, diluents, dispersing agents, suspending agents, thickening agents, and/or excipients described herein. The pharmaceutical composition facilitates administration of the compound to an organism.

As used herein, the terms "effective amount," "pharmaceutically effective amount," and "therapeutically effective amount" refer to an amount sufficient to provide desired biological results even if the substance is non-toxic. The results may be reduction and/or alleviation of signs, symptoms, or causes of a disease, or any other desired alteration of a biological system. The appropriate therapeutic amount in any individual case may be determined by a person of ordinary skill in the technical field through routine experimentation.

As used herein, the term "efficacy" may refer to the maximum effect (Emax) achieved by an analysis method.

The term "local administration" refers to administering a pharmaceutical ingredient to or near a patient’s muscle or subdermal location by a non-systemic route. Therefore, local administration excludes administration through systemic routes such as intravenous or oral administration.

The "therapeutic" treatment is treatment administered to a subject exhibiting signs or symptoms of pathology, with the aim of reducing or eliminating the signs or symptoms.

As used herein, the term "treatment" or "treating" is defined as applying or administering a therapeutic agent, i.e., the composition of the present invention (alone or in combination with other pharmaceutical agents), to a patient (e.g. for diagnostic or in vivo applications), to cure, heal, alleviate, relieve, alter, remedy, ameliorate, improve or affect the conditions contemplated herein, symptoms of the conditions contemplated herein, or the potential to develop into the conditions contemplated herein, or applying or administering a therapeutic agent to tissue or cell lines isolated from a patient (e.g., for diagnostic or in vitro applications). The conditions contemplated herein may either be symptoms of the conditions contemplated herein or have the potential to develop into the conditions contemplated herein. The treatment may be specifically tailored or modified based on knowledge gained from the field of pharmacology.

Scope: Throughout the present disclosure, various aspects of the present invention may be presented in the format of a range. The range values described in this specification comprise the boundary values, i.e., all values above the lower limit and below the upper limit, unless otherwise specified. It should be understood that the description in the format of a range is merely for convenience and brevity and should not be construed as an inflexible limitation on the scope of the invention. Accordingly, the description of a range should be considered as specifically disclosing not only the individual numerical values within the range, but also all possible subranges. For example, the description of a range such as 1 to 6 should be regarded as indicating the individual values within the range, such as 1, 2, 2.7, 3, 4, 5, 5.3 and 6, as well as subranges such as 1 to 3, 1 to 4, 1 to 5, 2 to 4, 2 to 6 and 3 to 6. This applies regardless of the width of the scope.

As used herein, ’%’ used in expressing the amount of the composition refers to a w/v% content unless otherwise specified, and refers to a w/v% value based on the entire composition unless otherwise specified.

As used herein, the symbol ’/’ in ’at least one selected from the group consisting of glycocholic acid or taurocholic acid and salts thereof/phosphatidylcholine’ indicates a fraction in a commonly used form.

## EFFECT OF THE INVENTION

The injectable composition for localized fat reduction of the present invention comprising taurocholic acid or glycocholic acid (or a salt thereof) and phosphatidylcholine (PPC) in a specific mixing ratio provides a safe and stable

formulation and is highly effective in reducing fat cells without various side effects, and may be administered to the affected area at shorter intervals to maximize the effects.

## BRIEF DESCRIPTION OF THE DRAWINGS

FIG. 1 shows an overview of the clinical trial design for determining the optimal dose of AYP-101.
FIG. 2 shows an overview of the clinical trial design for determining the administration interval of AYP-101.
FIG. 3 shows the results of comparison of the proportion of subjects whose submental fat improved by 1 grade or more as reported by clinicians and patients, depending on administration interval and administration dose.

## DETAILED DESCRIPTION OF THE EMBODIMENTS

Hereinafter, the present invention will be described in detail.
However, the following embodiments just exemplify the present invention and the scope of the present disclosure is not limited to the following embodiments.

(Embodiment)

### <Embodiment 1> Preparation of drug to be tested

AYP-101 was prepared by dissolving 25.0 mg or 50.0 mg of phosphatidyl choline (PPC) with glycocholic acid (GCA) in 1 mL and adding 9 mg (0.9%) of benzyl alcohol. The specific preparation process is as follows. Water for injection was added to the washed and sterilized preparation tank (at room temperature), and sodium hydroxide (0.04 to 0.72%) was added to the water for injection. Afterwards, glycocholic acid (GCA), benzyl alcohol and sodium chloride were added, stirred, and dissolved. Afterwards, phosphatidylcholine was added and stirred at 200 RPM under the light-shielded and sealed conditions, at room temperature (25°C), and under a nitrogen pressure. After completion of stirring, the pH was adjusted (if necessary, with additional 0.001 to 0.04% sodium hydroxide or 0.001 to 0.6% hydrochloric acid), filtered through a 0.2 μm filter, and then filled and sealed in vials.

### <Embodiment 2> Clinical trial - determination of optimal dose (4 week dosing interval)

2-1. Purpose of clinical trial

For non-surgical improvement of the appearance of adult patients with moderate to severe protruding or excessive submental fat by using the test drug (AYP-101) prepared in Embodiment 1, the test drug and the control drug (placebo for AYP-101) were administered to determine the optimal dose of the test drug and evaluate its safety and effectiveness.
For this purpose, a clinician (independent evaluator) and the subject evaluated the improvement in submental fat, administered the final clinical trial drug and checked whether the proportion of the subjects whose Clinician-Reported Submental Fat Rating Scale (CR-SMFRS) and Patient-Reported Submental Fat Rating Scale (PR-SMFRS), were reduced by 1 grade or more, as compared to the baseline value, in the test groups by dose was superior to that in the control group. This study was designed as Test Group 1 (AYP-101 25 mg/mL, 0.2 mL/Point), Test Group 2 (AYP-101 50 mg/mL, 0.2 mL/Point), Test Group 3 (AYP-101 50 mg/mL, 0.4 mL/Point) and control group (AYP-101 0 mg/mL, 0.2 mL/Point).

2-2. Clinical trial plan

This clinical trials were conducted by multicenter, randomized, double-blind and placebo-controlled studies. After the subjects who met the selection and exclusion criteria agreed in writing to participate in the clinical trial, the necessary examinations and tests were conducted according to the clinical trial protocol, and randomization was conducted only to those subjects whose suitability assessment results met the selection criteria. The randomized subjects were assigned to one of Test Group 1, Test Group 2, Test Group 3 and control group (1:1:1:1) and administered with the drug for clinical trials at 4-week intervals, at the grid interval of 1 cm, at 50 points (injection points), up to 6 times. The overall schedule of this clinical trial was as shown in FIG. 1.

2-3. Demographic information and other baseline characteristics

This clinical trial was conducted at a total of four institutions in Korea. A total of 219 subjects gave written consent and underwent screening, of which 65.30% (143/219) were randomly assigned. 37 people were assigned to Test Group 1 (AYP-101 25 mg/mL, 0.2 mL/Point), 34 people were assigned to Test Group 2 (AYP-101 50 mg/mL, 0.2 mL/Point), 35 people were assigned to Test Group 3 (AYP-101 50 mg/mL, 0.2 mL/Point), and 37 people were assigned to the control group (AYP-101 0 mg/mL, 0.2 mL/Point). 75.68% (28/37 people), 85.29% (29/34 people), 85.71% (30/35 people), and 75.68% (28/37 people) of the subjects, respectively, in the groups completed the test.

[Table 1]

| | AYP-101 2.5% (5 mg/cm$^2$) (n=37) | AYP-101 5.0% (10 mg/cm$^2$) (n=33) | AYP-101 5.0% (20 mg/cm$^2$) (n=35) | Placebo of AYP-101 (0 mg/cm$^2$) (n=37) |
|---|---|---|---|---|
| Age Mean±SD | 40.27±13.05 | 38.91±12.47 | 36.29±11.62 | 35.38±10.23 |
| Age min, max | 20.00, 63.00 | 21.00, 61.00 | 23.00, 64.00 | 19.00, 50.00 |
| Gender, n(%) | | | | |
| Male | 14 (37.84) | 6 (18.18) | 11 (31.43) | 13 (35.14) |
| Female | 23 (62.16) | 27 (81.82) | 24 (68.57) | 24 (64.86) |
| Height Mean ±SD (min, max) | 165.76±9.07 (149.90,182.20) | 164.11±7.30 (155.00, 187.60) | 163.55±8.30 (149.50, 178.40) | 166.51±8.90 (152.70, 186.60) |
| Weight Mean ±SD (min, max) | 71.62±13.81 (52.70, 104.20) | 70.03±11.53 (50.80, 93.10) | 67.00±12.46 (47.50, 90.20) | 70.10±18.04 (47.80, 111.50) |
| BMI Mean±SD (min, max) | 25.88±3.24 (21.10, 34.00) | 25.91±3.28 (20.00, 32.80) | 24.96±3.79 (19.70, 34.60) | 24.94±4.16 (18.80, 34.40) |
| Presence of disease at present, n(%) | | | | |
| Yes | 9 (24.32) | 9 (27.27) | 9 (25.71) | 10 (27.03) |
| No | 28 (75.68) | 24 (72.73) | 26 (74.29) | 27 (72.97) |
| Presence of preceding drug, n(%) | | | | |
| Yes | 0 (0.00) | 4 (12.12) | 3 (8.57) | 2 (5.41) |
| No | 37 (100.00) | 29 (87.88) | 32 (91.43) | 35 (94.59) |
| Presence of drug in combination, n(%) | | | | |
| Yes | 17 (45.95) | 10 (30.30) | 20 (57.14) | 12 (32.43) |
| No | 20 (54.05) | 23 (69.70) | 15 (42.86) | 25 (67.57) |

2-4. Results of evaluation of efficacy

The main endpoint was analyzed for 110 patients in the FAS (Full Analysis Set). At 12 weeks after the final administration, subjects whose main endpoints, CR-SMFRS and PR-SMFRS, improved by more than 1 grade, as compared to the baseline value, were 42.31% (11/26 people) in Test Group 1, 46.15% (12/26 people) in Test Group 2, and 40.00% (12/30 people) in Test Group 3. That is, statistical significance was confirmed in all test groups (P=0.0081, P=0.0037 and P=0.0109), as compared to the control group showing 10.71% (3/28 people). This meant that the test drug AYP-101 was effective in reducing submental fat.

[Table 2]

| Proportion of subjects whose CR-SMFRS & PR-SMFRS improved by 1 grade or more at 12 weeks after final administration | | | |
|---|---|---|---|
| AYP-101 Placebo (0 mg/cm$^2$) | AYP-101 2.5% (5 mg/cm$^2$) 25mg/mL / 0.2mL/inj. | AYP-101 5.0% (10 mg/cm$^2$) 50mg/mL / 0.2mL/inj. | AYP-101 5.0% (20 mg/cm$^2$) 50mg/mL / 0.4mL/inj. |
| 10.71% (3/28) | 42.31% (11/26) | 46.15% (12/26) | 40.00% (12/30) |

Secondary endpoint analysis conducted to obtain additional clinical study information confirmed that the proportion of subjects whose CR-SMFRS improved by 1 grade or more at 12 weeks after the final dose was statistically significant in all test groups, as compared to the control group.

[Table 3]

| Proportion of subjects whose CR-SMFRS improved by 1 grade or more at 12 weeks after final administration | | | |
|---|---|---|---|
| AYP-101 Placebo (0 $mg/cm^2$) | AYP-101 2.5% (5 $mg/cm^2$) 25mg/mL / 0.2mL/inj. | AYP-101 5.0% (10 $mg/cm^2$) 50mg/mL / 0.2mL/inj. | AYP-101 5.0% (20 $mg/cm^2$) 50mg/mL / 0.4mL/inj. |
| 14.29% (4/28) | 65.38% (17/26) | 65.38% (17/26) | 53.33% (16/30) |

This clinical trial confirmed that the effect of reducing submental fat in the AYP-101 groups at all doses was superior, as compared to the control group. In addition, it was confirmed that a concentration of 5 or 10 $mg/cm^2$ might be more effective than SPC 20 $mg/cm^2$, showing that a higher concentration does not guarantee a better submental fat improvement effect. In addition, since the maximum severity of pain and some adverse events or drug adverse events tended to be somewhat high, it was considered that 20 $mg/cm^2$ was not the optimal dose.

2-5. Results of evaluation of medication compliance

In the study on administration of AYP-101 at 4-week intervals, the medication compliance of both test subjects and subjects who terminated the clinical trial early was above 99.0% on average in all AYP-101 groups and control groups, showing no difference. There was no statistically significant difference between the groups in the number of administrations of clinical trial drugs and the total number of administrations. The total dose (mL) and total dose (mg) of the clinical trial drug differed between the groups depending on the administered dose and the SPC concentration of AYP-101.

[Table 4]

| | AYP-101 2.5% (5 $mg/cm^2$) (n=37) | AYP-101 5.0% (10 $mg/cm^2$) (n=33) | AYP-101 5.0% (20 $mg/cm^2$) (n=35) | Placebo of AYP-101 (0 $mg/cm^2$) (n=37) |
|---|---|---|---|---|
| Medication compliance (%) | | | | |
| Mean±SD | 99.10±3.82 | 99.49±2.90 | 100.00±0.00 | 100.00±0.00 |
| Min, Max | 83.33, 100.00 | 83.33, 100.00 | 100.00, 100.00 | 100.00, 100.00 |
| Number of administrations of drugs for clinical trials, n (%) | | | | |
| 1 time | 1 (2.70) | 1 (3.03) | 1 (2.86) | 4 (10.81) |
| 2 times | 2 (5.41) | 1 (3.03) | 2 (5.71) | 4 (10.81) |
| 3 times | 1 (2.70) | 0 (0.00) | 0 (0.00) | 0 (0.00) |
| 4 times | 2 (5.41) | 3 (9.09) | 2 (5.71) | 1 (2.70) |
| 5 times | 4 (10.81) | 1 (3.03) | 1 (2.86) | 0 (0.00) |
| 6 times | 27 (72.97) | 27 (81.82) | 29 (82.86) | 28 (75.68) |
| Total number of administrations of drugs for clinical trials | | | | |
| Mean±SD | 5.35±1.32 | 5.52±1.20 | 5.49±1.29 | 4.97±1.89 |
| Min, Max | 1.00, 6.00 | 1.00, 6.00 | 1.00, 6.00 | 1.00, 6.00 |
| Total dose of drugs for clinical trials (mL) | | | | |
| Mean±SD | 48.63±12.98 | 50.83±12.52 | 100.46±26.06 | 45.18±18.66 |
| Min, Max | 8.80, 60.00 | 10.00, 60.00 | 20.00, 120.00 | |
| Total dose of drugs for clinical trials (mg) | | | | |
| Mean±SD | 1,215.68±324.60 | 2,541.52±625.87 | 5,022.86±1,302.85 | 0.00±0.00 |
| Min, Max | 220.00, 1,500.00 | 500.00, 3,000.00 | 1,000.00, 6,000.00 | 0.00, 0.00 |

2-6. Results of evaluation of safety evaluation

The occurrence rate of adverse events and local drug adverse events was 100.00% in all test groups and control group. The occurrence rate of systemic adverse events was 5.41% (2/37 people, 2 cases) in Test Group 1, 6.06% (2/33 people, 2 cases) in Test Group 2, and 17.14% (6/35 people, 8 cases) in test group 3), and 5.41% (2/37 people, 2 cases) in the control group. There was no statistically significant difference between in the groups in the occurrence rate of systemic adverse events (P=0.2754). The occurrence rate of systemic adverse drug events was 2.86% (1/35 people, 1 case) in Test Group 3, and no systemic adverse drug events occurred in Test Group 1, Test Group 2, and control group. There was no statistically significant difference between the groups in the occurrence rate of systemic adverse events (P=0.4789). The occurrence rate of other adverse events was 29.73% (11/37 people, 23 cases) in Test Group 1, 36.36% (12/33 people, 21 cases) in Test Group 2, and 45.71% (16/35 people, 34 cases) in Test Group 3. The occurrence rate of other adverse events in the control group was 32.43% (12/37 people, 22 cases). There was no statistically significant difference between the groups in the occurrence rate of other adverse events (P=0.5177). The occurrence rate of other adverse drug reactions was 6.06% (2/33 people, 2 cases) in Test Group 2, 8.57% (3/35 people, 4 cases) in Test Group 3, and no other adverse drug reactions occurred in Test Group 1. The occurrence rate of other adverse drug reactions in the control group was 2.70% (1/37 people, 1 case). There was no statistically significant difference between the groups in the occurrence rate of other adverse drug reactions (P=0.2396). The occurrence rate of other serious adverse events was 2.70% (1/37 people, 1 case) in Test Group 1, and no other serious adverse events occurred in Test Groups 2 and 3. The occurrence rate of other serious adverse events in the control group was 2.70% (1/37 people, 1 case). There was no statistically significant difference between the groups in the occurrence rate of other serious adverse events (P=1.0000). The local serious adverse events, local serious adverse drug reactions, dropout due to local adverse events, systemic serious adverse drug reactions, dropout due to systemic adverse events, other serious adverse drug reactions, and dropout due to other adverse events did not occur in this clinical trial.

The status of occurrence of adverse events is presented in Table 5, and the number of occurrences of adverse events by tested item is presented in Table 6.

In the 37 people in Test Group 1, the occurrence rate of local adverse events was 100.00% (37/37 people, 1,190 cases) and, in more detail, was highest in the order of 100.00% (37/37 people, 183 cases) for 'pain', 100.00% (37/37 people, 1,190 cases) for 'local side effects', 91.89% (34/37 people, 147 cases) for 'edema', and 81.08% (30/37 people, 129 cases) for 'skin pulling.' In the 33 people in Test Group 2, the occurrence rate of local adverse events was 100.00% (33/33 people, 1,035 cases), and, in more detail, was highest in the order of 100.00% (33/33 people, 172 cases, 130 cases) each for 'pain' and 'local swelling,' 87.88% (29/33 people, 86 cases) for 'hematoma (bruise),' and 81.82% (27/33 people, 106 cases) for 'heat sensation.' In the 35 patients in Test Group 3, the occurrence rate of local adverse events was 100.00% (35/35 people, 1,204 cases) and, in more detail, was highest in the order of 100.00% (35/35 people, 185 cases) for 'pain,' 97.14% (34/35 people, 134 cases) for 'skin pulling,' and 94.29% (33/35 people, 147 cases) for 'local edema.' In the 37 people in the control group, the occurrence rate of local adverse events was 100.00% (37/37 people, 820 cases) and, in more detail, 94.59% (35/37 people, 144 cases) for 'pain,' 72.97% (27/37 people, 107 cases) for 'local edema,' and 67.57% (25/37 people, 80 cases) for 'hematoma (bruise).' As a result of classifying the occurrence status of local adverse events into 5 duration periods of '+1 day,' '+3 days,' '+5 days,' '+7 days,' and '+10 days', there was no significant difference between the groups in the adverse event occurrence rate. However, as the duration increased, the number of adverse events tended to decrease.

As a result of analyzing the severity of all local adverse drug reactions excluding pain, it was 1.02 $\pm$ 0.14 points in Test Group 1, 1.07 $\pm$ 0.27 points in Test Group 2, and 1.09 $\pm$ 0.31 points in Test Group 3. As a result of analyzing the severity of all local adverse drug reactions except pain in the control group, it was 1.10 $\pm$ 0.31. It was statistically significant in Test Group 1, as compared to the control group (P<0.0001), but Test Groups 2 and 3 showed no statistically significant difference (P=0.0088, 0.1777). As a result of analyzing the maximum severity of all local adverse drug reactions excluding pain in Test Group 1, it was highest in the order of 67.57% (25/37 people) for 'mild,' and 29.73% (11/37 people) for 'moderate,' and no 'severe' local adverse drug reactions occurred. In test group 2, the maximum severity was highest in the order of 51.52% (17/33 people) for 'mild,' 45.45% (15/33 people) for 'moderate,' and 3.03% (1/33 people) for 'severe.' In Test Group 3, the maximum severity was highest in the order of 48.57% (17/35 people) for 'mild,' 37.14% (13/35 people) for 'moderate,' and 14.29% (5/35 people) for 'severe.' As a result of analyzing the occurrence rate of local adverse drug reactions according to severity in the control group, it was highest in the order of 59.46% (22/37 people) for 'mild,' 24.32% (9/37 people) for 'severe,' and 5.41% (2/37 people) for 'moderate.'

In the 37 people in Test Group 1, the occurrence rate of systemic adverse events was 5.41% (2/37 people, 2 cases) and, in more detail, it was 2.70% (1/37 people, 1 case each) each for 'upper respiratory tract infection' and 'epidemic cold.' In the 33 people in Test Group 2, the occurrence rate of systemic adverse events was 6.06% (2/33 people, 2 cases) and, in more detail, it was 3.03% (1/33 people, 1 case each) each for 'nasopharyngitis' and 'epidemic cold.' In the 35 people in Test Group 3, the occurrence rate of systemic adverse events was 17.17% (6/35 people, 8 cases) and, in more detail, it was highest in the order of 5.71% (2/35 people, 2 cases) for 'epidemic cold,' 2.86% (1/35 people, 3 cases, 1 case, 1 case)

each for ’headache’, ’nasopharyngitis’ and ’upper respiratory infection.’ In the 37 people in the control group, the occurrence rate of systemic adverse events was 5.41% (2/37 people, 2 cases) and, in more detail, it was 5.41% (2/37 people, 2 cases) for ’upper respiratory tract infection.’

There were no adverse drug reactions in the 37 patients in Test Group 1. The rate of adverse drug reactions in the 33 people in Test Group 2 was 6.06% (2/33 people, 2 cases). As a result of classifying adverse drug reactions into "SOC" and "PT," it was 3.03% (1/33 people, 1 case) each for "Dermal cyst" in "Skin and subcutaneous tissue disorders," and "Blood creatine phosphokinase increased" in "Investigations." In the 35 patients in Test Group 3, the occurrence rate of adverse drug reactions was 8.57% (3/35 patients, 4 cases). As a result of classifying adverse drug reactions by SOC, it was in the following order 5.71% (2/35 people, 2 cases) for ‘Skin and subcutaneous tissue disorders,’ 2.86% (1/ 35 people, 1 case) each for ‘Gastrointestinal disorders’ and ‘Nervous system disorders.’ As a result of classifying adverse drug reactions into PT, the occurrence rate was 2.86% (1/35 people, 1 case) each for ’Erythema’, ’Pigmentation disorder’, and ’Vomiting.’ In the 37 people in the control group, the adverse drug reaction rate was 2.70% (1/37 people, 1 case). As a result of classifying adverse drug reactions into "SOC" and "PT," it was 2.70% (1/37 people, 1 case) for "Rash" in "Skin and subcutaneous tissue disorders."

In the 37 people in Test Group 1, the occurrence rate of serious adverse events was 2.70% (1/37 people, 1 case). As a result of classifying serious adverse events into "SOC" and "PT," the occurrence rate of serious adverse events was 2.70% (1/37 people, 1 case) for "Hand fracture" in "Injury, poisoning and procedural complications." No serious adverse events occurred in 33 people in Test Group 2 and 35 people in Test Group 3, and the occurrence rate of serious adverse events in the 37 people in the control group was 2.70% (1/37 people, 1 case). As a result of classifying serious adverse events into "SOC" and "PT," the occurrence rate of serious adverse events was 2.70% (1/37 people, 1 case) for "Meningitis" in "Infections and infestations."

In the clinical laboratory test evaluation, the number of individual clinically significant abnormalities was 71 in 27 people. There were 17 cases in 7 people in Test Group 1, 12 cases in 6 people in Test Group 2, 15 cases in 7 people in Test Group 3, and 27 cases in 7 people in the control group. In more detail, the number of clinically significant (CS) abnormalities in Test Group 1 was highest in the order of 6 cases for ’ALT’, 4 cases each for ’AST’ and ’Creatine kinase,’ and 1 case each for ’CRP’ and ’r-GT’ and ‘TG.’ In Test Group 2, the number of CS abnormalities was highest in the order of 5 cases for ’Creatine kinase,’ 3 cases each for ’TG,’ and 2 cases each for ’ALT’ and ’r-GT.’ In Test Group 3, the number of CS abnormalities was highest in the order of 3 cases for ’ALT,’ 2 cases each for ’CRP,’ ’ESR’ and ’Glucose,’ and 1 case each for ’AST’, ’HbA1c’ and ’r-GT.’ In more detail, the number of clinically significant abnormalities (CSR) in the control group were highest in the order of 8 cases for ’TG,’ 6 cases each for ’ALT’ and ’r-GT,’ and 2 cases each for ’AST’ and ’Uric acid.’

[Table 5]

| | AYP-101 2.5% (5 mg/cm$^2$) (n37) n(%), [Number of occurrence] | AYP-101 5.0% (10 mg/cm$^2$) (n=33) n(%), [Number of occurrence] | AYP-101 5.0% (20 mg/cm$^2$) (n=35) n(%), [Number of occurrence] | Placebo of AYP-101 (0 mg/cm$^2$) (n=37) n(%), [Number of occurrence] |
|---|---|---|---|---|
| Adverse events | | | | |
| Local | 37 (100.00),[1190] | 33 (100.00),1035] | 35 (100.00),[1204] | 37 (100.00),[820] |
| Systemic | 2 (5.41),[2] | 2 (6.06),[2] | 6 (17.14),[8] | 2 (5.41),[2] |
| Other | 11 (29.73) | 12 (36.36) | 16 (45.71) | 12 (32.43) |
| Adverse drug reactions | | | | |
| Local | 37 (100.00),[1180] | 33 (100.00),[1018] | 35 (100.00),[1186] | 37 (100.00),[799] |
| Systemic | 0 (0.00),[0] | 0 (0.00),[0] | 1 (2.86),[1] | 0 (0.00),[1] |
| Other | 0 (0.00),[0] | 2 (6.06),[2] | 3 (8.57),[4] | 1 (2.70),[1] |
| Serious adverse events | | | | |
| Local | 0 (0.00),[0] | 0 (0.00),[0] | 0 (0.00),[0] | 0 (0.00),[0] |
| Systemic | 0 (0.00),[0] | 0 (0.00),[0] | 0 (0.00),[0] | 0 (0.00),[0] |
| Other | 1 (2.70),[1] | 0 (0.00),[0] | 0 (0.00),[0] | 1 (2.70),[1] |
| Serious adverse drug reactions | | | | |
| Local | 0 (0.00),[0] | 0 (0.00),[0] | 0 (0.00),[0] | 0 (0.00),[0] |
| Systemic | 0 (0.00),[0] | 0 (0.00),[0] | 0 (0.00),[0] | 0 (0.00),[0] |

(continued)

| Serious adverse drug reactions | | | | |
|---|---|---|---|---|
| Other | 0 (0.00),[0] | 0 (0.00),[0] | 0 (0.00),[0] | 0 (0.00),[0] |

[Table 6]

| | AYP-101 2.5% (5 mg/cm$^2$) (n37) n(%), [Number of occurrence] | AYP-101 5.0% (10 mg/cm$^2$) (n=33) n(%), [Number of occurrence] | AYP-101 5.0% (20 mg/cm$^2$) (n=35) n(%), [Number of occurrence] | Placebo of AYP-101 (0 mg/cm$^2$) (n=37) n(%), [Number of occurrence] |
|---|---|---|---|---|
| Local adverse drug reactions | 37 (100.0), [2421] | 33 (100.0), [2161] | 35 (100.0), [2504] | 37 (100.0), [1655] |
| Severity | | | | |
| Mean±SD | 1.02±0.14 | 1.07±0.27 | 1.09±0.31 | 1.10±0.31 |
| Median | 1.0 | 1.0 | 1.0 | 1.0 |
| Min, Max | 1.00, 2.00 | 1.00, 3.00 | 1.00, 3.00 | 1.00, 3.00 |
| Maximum severity | | | | |
| Mild | 36 (97.3), [1853] | 33 (100.0), [1559] | 35 (100.0), [1863] | 33 (89.2), [1222] |
| Moderate | 11 (29.7), [39] | 17 (51.5), [117] | 18 (51.4), [154] | 11 (29.7), [127] |
| Severe | 0 (0.0), [0] | 1 (3.0), [2] | 5 (14.3), [15] | 3 (8.1), [3] |
| Results | | | | |
| Recovered | 37 (100.0), [2475] | 33 (100.0), [2248] | 35 (100.0), [2598] | 37 (100.0), [1719] |
| Recovering | 5 (13.5), [8] | 0 (0.0), [0] | 4 (11.4), [6] | 6 (16.2), [18] |
| Recovered but showing aftereffects | 1 (2.7), [1] | 0 (0.0), [0] | 1 (2.9), [1] | 0 (0.0), [0] |
| Dead due to adverse drug reactions | 0 (0.0), [0] | 0 (0.0), [0] | 0 (0.0), [0] | 0 (0.0), [0] |
| Unknown (Tracking failed) | 0 (0.0), [0] | 0 (0.0), [0] | 0 (0.0), [0] | 1 (2.70), [1]* |
| Causal relationship with drugs for clinical trials | | | | |
| Relevant - certain | 21 (56.76), [898] | 19 (57.58), [587] | 22 (62.86), [1089] | 17 (45.95), [809] |
| Relevant - fairly certain | 11 (29.73), [231] | 11 (33.33), [175] | 11 (31.43), [265] | 11 (29.73), [100] |
| Relevant - possible | 20 (54.05), [1292] | 19 (57.58), [1401] | 21 (60.00), [1155] | 21 (56.76), [747] |
| Relevant - difficult to evaluate | 0 (0.0), [0] | 0 (0.0), [0] | 0 (0.0), [0] | 0 (0.0), [0] |
| Relevant - cannot be evaluated | 0 (0.0), [0] | 0 (0.0), [0] | 0 (0.0), [0] | 0 (0.0), [0] |
| Not relevant - less likely | 17 (45.95), [63] | 14 (42.42), [85] | 19 (54.29), [97] | 16 (43.24), [82] |

(continued)

| Measures for drugs for clinical trials | | | | |
|---|---|---|---|---|
| Administration stopped | 0 (0.0), [0] | 0 (0.0), [0] | 0 (0.0), [0] | 0 (0.0), [0] |
| Dosage decreased | 0 (0.0), [0] | 0 (0.0), [0] | 0 (0.0), [0] | 0 (0.0), [0] |
| Dosage increased | 0 (0.0), [0] | 0 (0.0), [0] | 0 (0.0), [0] | 0 (0.0), [0] |
| Dosage unchanged | 37 (100.0), [2484] | 33 (100.0), [2248] | 35 (100.0), [2606] | 37 (100.0), [1738] |
| Uncertain | 0 (0.0), [0] | 0 (0.0), [0] | 0 (0.0), [0] | 0 (0.0), [0] |
| None | 0 (0.0), [0] | 0 (0.0), [0] | 0 (0.0), [0] | 0 (0.0), [0] |
| Measures other than drugs for clinical trials | | | | |
| No action | 37 (100.0), [2462] | 33 (100.0), [2220] | 35 (100.0), [2536] | 37 (100.0), [1730] |
| Drug treatment | 8 (21.6), [12] | 6 (18.2), [15] | 16 (45.7), [38] | 7 (18.9), [8] |
| Non-drug treatment | 1 (2.7), [8] | 2 (6.1), [12] | 4 (11.4), [30] | 0 (0.0), [0] |
| Drug/non-drug treatment | 2 (5.4), [2] | 1 (3.0), [1] | 1 (2.9), [2] | 0 (0.0), [0] |
| Duration | | | | |
| 1-3 days | | | | |
| 4-7 days | | | | |
| 8-14 days | | | | |
| 15-30 days | | | | |
| 31-60 days | | | | |
| >60 days | | | | |
| *Abandoned visit to institution due to worsening meningitis<br>%: n / (number of safety test subjects in each assigned group) x 100<br>n(%): The number of test subjects is double counted for each item.<br>Number of occurrence %: (Number of occurrences of each item/Number of occurrences of each assigned group) × 100Severity: mild (1), moderate (2) and severe (3), excluding local adverse event pain | | | | |

**<Embodiment 3> Clinical trial - determination of dosing intervals**

3-1. Purpose of clinical trial

Using the test drug (AYP-101) prepared in Embodiment 1 for non-surgical improvement of the appearance of adult patients with moderate to severe protruding or excessive submental fat (placebo of AYP-101), the test drug and the control drug (AYP-101) were administered at two-week intervals, the administration interval and optimal dose were determined, and safety and effectiveness were evaluated.

For this purpose, researchers and subjects participating in clinical trials evaluated, and checked whether at 4 weeks after the final administration of the drug for clinical trials, the proportion of the subject in the test groups by dose whose Estimator-Reported Submental Fat Rating Scale (ER-SMFRS) and Subject-Reported Submental Fat Rating Scale (SR-SMFRS), were reduced by 1 grade or more, as compared to the baseline value, was superior to that in the control group.

3-2. Clinical trial plan

After the subjects who met the selection and exclusion criteria agreed in writing to participate in the clinical trial,

the necessary examinations and tests were conducted according to the clinical trial protocol, and randomization was conducted only to those subjects whose suitability assessment results met the selection criteria. The randomized subjects were assigned to one of Test Group 1 (AYP-101 25 mg/mL), Test Group 2 (AYP-101 50 mg/mL) and control group (placebo of (AYP-101) (1:1:1) and administered with the drug for clinical trials at 2-week intervals, at the grid interval of 1 cm, at 50 points (injection points), up to 6 times. The overall schedule of this clinical trial was as shown in FIG. 2.

3-3. Demographic and other baseline characteristics

The average age (only) of the subjects contained in SS was 38.82 ± 10.96 years for Test Group 1, 41.03 ± 8.97 years for Test Group 2, and 42.63 ± 11.16 years for the control group, and there was no statistically significant difference between the test groups and the control group (p of Test Group 1 vs. control group was 0.1700; and p of Test Group 2 vs. control group was 0.5354). As for gender, in Test Group 1, male was 21.21% (7/33 people) and female was 78.79% (26/33 people). In Test Group 2, male was 19.35% (6/31 people) and female was 80.65% (25/31 people). In the control group, male was 21.88% (7/32 people) and female was 78.13% (25/32 people), and there was no statistically significant difference between the test groups and the control group (p of Test Group 1 group vs. control group was 0.9482; and p of Test Group 2 vs. control group was 0.8048). The average weight was 65.93±13.18 kg for Test Group 1, 66.31±12.06 kg for Test Group 2, and 66.26±14.42 kg for the control group, and there was no statistically significant difference between the test groups and the control group (p of Test Group 1 vs. control group was 0.7133; and p of Test Group 2 vs. control group was 0.8961). The average BMI was 24.22±3.59 kg/$m^2$ for Test Group 1, 24.74±3.63 kg/$m^2$ for Test Group 2, 24.50±4.05 kg/m2 for the control group, and there was no statistically significant difference between the test groups and the control group (p of Test Group 1 vs. control group was 0.7748; and p of Test Group 2 vs. control group was 0.8040).

[Table 7]

| | AYP-101 2.5% (5 mg/$cm^2$) (n=33) | AYP-101 5.0% (10 mg/$cm^2$) (n=31) | Placebo of AYP-101 (0 mg/$cm^2$) (n=32) |
|---|---|---|---|
| Age Mean±SD | 38.8±11.0 | 41.0±9.0 | 42.6±11.2 |
| (min-max) | 21.0-60.0 | 27.0-62.0 | 20.0-61.0 |
| Gender, n(%) | | | |
| Male | 7 (21.2) | 6 (19.4) | 7 (21.9) |
| Female | 26 (78.8) | 25 (80.7) | 25 (78.1) |
| Height (cm) Mean±SD (min-max) | 164.4±6.5<br>151.0-175.6 | 163.4±6.4<br>149.8-179.3 | 164.0±8.3<br>151.9-183.5 |
| Weight (kg) Mean±SD (min-max) | 65.9±13.2<br>38.7-101.0 | 66.3±12.1<br>49.0-96.0 | 66.3±14.4<br>42.1-100.0 |
| BMI Mean±SD (min-max) | 24.2±3.6<br>16.2-32.8 | 24.7±3.6<br>19.6-34.4 | 24.5±4.1<br>18.0-32.6 |
| Whether to be vaccinated against COVID-19, n(%) | | | |
| Yes | 32 (97.0) | 30 (96.8) | 32 (100.0) |
| No | 1 (3.03) | 1 (3.23) | 0 (0.00) |
| The presence of disease at present, n(%) | | | |
| Yes | 10 (30.3) | 9 (29.0) | 9 (28.1) |
| No | 23 (69.7) | 22 (71.0) | 23 (71.9) |
| The presence of preceding drugs, n(%) | | | |
| Yes | 10 (30.3) | 11 (35.5) | 14 (43.8) |
| No | 23 (69.7) | 20 (64.5) | 18 (56.3) |
| The presence of drugs in combination, n(%) | | | |
| Yes | 17 (51.5) | 8 (25.8) | 11 (34.4) |

(continued)

| The presence of drugs in combination, n(%) | | | |
|---|---|---|---|
| No | 16 (48.5) | 23 (74.2) | 21 (65.6) |

3-4. Results of evaluation of effectiveness

In the main analysis group, FAS (Full Analysis Set), the proportion of subjects whose main endpoints, ER-SMFRS and SR-SMFRS, were reduced by 1 grade or more, as compared to the baseline value, at 4 weeks after the final administration was evaluated, and the proportion was 69.70% (23/33 people) for Test 1 Group, 48.39% (15/31 people) for Test 2 Group, 22.58% (7/31 people) for the control group, and both Test Group 1 and Test 2 showed a greater effect of reducing submental fat, as compared to the control group. The statistical significance based on the multiple test set up in this study was found to be significant at p=0.0002 in Test Group 1, confirming that when AYP-101 is administered at 2-week intervals, the optimal dose is that in Test Group 1 (AYP-101 25mg/mL).

[Table 8] Proportion of improvement in ER-SMFRS & SR-SMFRS by 1 grade or more at 4 weeks after final administration

| Placebo of AYP-101 | AYP-101 2.5% (5 mg/cm$^2$)<br>25mg/mL / 0.2mL/inj. | AYP-101 5.0% (10 mg/cm$^2$)<br>50mg/mL / 0.2mL/inj. |
|---|---|---|
| 22.58% (7/31) | 69.70% (23/33) | 48.39% (15/31) |

Regarding the proportion of subjects whose Clinician-Reported Submental Fat Rating Scale (CR-SMFRS) and the Patient-Reported Submental Fat Rating Scale (PR-SMFRS), were reduced by 1 grade or more, as compared to the baseline value, the results of Embodiment 2 (see Table 2) and the results of Embodiment 3 (see Table 8) with an administration interval of 4 weeks were compared, which is shown in FIG. 3.

As a result of analyzing the proportion of subjects whose ER-SMFRS and SR-SMFRS, which are secondary endpoints conducted to obtain additional clinical study information, were reduced by 1 grade or more, as compared to the baseline value, at 12 weeks after the final administration, a statistically significant effect of reducing submental fat was shown in Test Group 1, as compared to the control group, confirming that the effect of reducing submental fat was maintained at 4 and 12 weeks after the final administration.

In addition, the proportion of subjects whose ER-SMFRS improved by 1 grade or more, as compared to the baseline value at 4 weeks after the final administration was 69.70% (23/33 people) in Test Group 1, 54.84% (17/31 people) in Test Group 2, and 25.81% (8/31 people) in the control group. At 12 weeks, the proportion was 72.73% (24/33 people) in Test Group 1, 58.06% (18/31 people) in Test Group 2, and 25.81% (8/31 people) was in the control group. As such, a statistically significant effect of reducing submental fat was confirmed in the test groups at all doses, as compared to the control group.

[Table 9]

| The proportion of subjects whose ER-SMFRS improved by 1 grade or more at 4 weeks after final administration | | |
|---|---|---|
| Placebo of AYP-101 | AYP-101 2.5% (5 mg/cm$^2$)<br>25mg/mL / 0.2mL/inj. | AYP-101 5.0% (10 mg/cm$^2$)<br>50mg/mL / 0.2mL/inj. |
| 25.81% (8/31) | 69.70% (23/33) | 54.84% (17/31) |

In addition, the proportion of subjects whose ER-SMFRS improved by 1 grade or more, as compared to the baseline value was 75.76% (25/33 people) in Test Group 1, 54.84% (17/31 people) in Test Group 2, and 29.03% (9/31 people) in the control group at 4 weeks after the final administration. At 12 weeks, 66.67% (22/33 people) in Test Group 1, 48.39% (15/31 people) in Test Group 2, and 25.81% (8/31 people) in the control group. That is, a statistically significant effect of reducing submental fat was confirmed in Test Group 1, as compared to the control group.

[Table 10]

| The proportion of subjects whose SR-SMFRS improved by 1 grade or more at 4 weeks after final administration | | |
|---|---|---|
| Placebo of AYP-101 | AYP-101 2.5% (5 mg/cm$^2$)<br>25mg/mL / 0.2mL/inj. | AYP-101 5.0% (10 mg/cm$^2$)<br>50mg/mL / 0.2mL/inj. |
| 29.03% (9/31) | 75.76% (25/33) | 54.84% (17/31) |

3-5. Results of evaluating medication compliance

In the study on the administration of AYP-101 at 2-week intervals, those who showed medication compliance of less than 67% (with the total number of administrations of less than 4 times) was only 1 person (1.04%) in the control group. There was no statistically significant difference between the test groups and the control group in the average number of administrations, average number of administration points and average dose of the total drugs for clinical trials (see Table 11).

[Table 11]

| | AYP-101 2.5% (5 mg/cm$^2$) (n=33) | AYP-101 5.0% (10 mg/cm$^2$) (n=31) | Placebo of AYP-101 (0 mg/cm$^2$) (n=32) |
|---|---|---|---|
| Total number of administrations of drugs for clinical trial (times) | | | |
| n | 33 | 31 | 32 |
| Mean±Std | 5.82±0.53 | 5.77±0.56 | 5.53±0.95 |
| Median | 6.00 | 6.00 | 6.00 |
| Min, Max | 4.00, 6.00 | 4.00, 6.00 | 2.00, 6.00 |
| p-value[b] | 0.1711W | 0.3400W | |
| Number of administration points of drugs for clinical trial (points) | | | |
| n | 33 | 31 | 32 |
| Mean±Std | 280.82±28.95 | 281.32±28.75 | 273.50±46.68 |
| Median | 290.00 | 294.00 | 300.00 |
| Min, Max | 190.00, 300.00 | 190.00, 300.00 | 100.00, 300.00 |
| p-value[b] | 0.6392W | 0.6337W | |
| Dosage of drugs for clinical trial (ml) | | | |
| n | 33 | 31 | 32 |
| Mean±Std | 56.16±5.79 | 56.26±5.75 | 54.70±9.34 |
| Median | 58.00 | 58.80 | 60.00 |
| Min, Max | 38.00, 60.00 | 38.00, 60.00 | 20.00, 60.00 |
| p-value[b] | 0.6392W | 0.6337W | |
| T: Two sample t-test / W: Wilcoxon's rank sum test / NA: Not applicable<br>p-value[b]: Test between treatment groups | | | |

3-6. Results of evaluating safety

In the study on the administration of AYP-101 at 2-week intervals, the occurrence rate of adverse events was 100.00% (33/33 people, 532 cases) in Test Group 1, 96.77% (30/31 people, 538 cases) in Test Group 2, and 62.50% (20//32 people, 180 cases) in the control group. There was a statistically significant difference between the test groups and the control group (p of Test Group 1 vs. control group was less than 0.0001, p of Test Group 2 vs. control group was 0.0008). The occurrence rate of local adverse events was 100.00% (33/33 people, 525 cases) in Test Group 1, 96.77% (30/31 people, 529 cases) in Test Group 2, and 50.00% (16/32 people, 172 cases) in the control group. There was a statistically significant difference between the test groups and the control group (each $p<0.0001$). The occurrence rate of systemic

adverse events was 3.03% (1/33 people, 1 case) in Test Group 1, 9.68% (3/31 people, 3 cases) in Test Group 2, and 6.25% (2/32 people, 2 cases) in the control group. There was no statistically significant difference between the test groups and the control group (p of Test Group 1 vs. control group was0.6132; and p of Test Group 2 vs. control group was 0.6719). The occurrence rate of other adverse events was 18.18% (6/33 people, 6 cases) in Test Group 1, 16.13% (5/31 people, 6 cases) in Test Group 2, and 18.75% (6/32 people, 6 cases) in the control group. There was no statistically significant difference between the test groups and the control group (p of Test Group 1 vs. control group was 0.9529; and p of Test Group 2 vs. control group was 0.7841).

The occurrence rate of adverse drug reactions was 100.00% (33/33 people, 525 cases) in Test Group 1, 96.77% (30/31 people, 532 cases) in Test Group 2, and 50.00% (16/32 people, 172 cases) in the control group. There was a statistically significant difference between the test groups and the control group (each p<0.0001). The occurrence rate of local adverse drug reactions was 100.00% (33/33 people, 525 cases) in Test Group 1, 96.77% (30/31 people, 529 cases) in Test Group 2, and 50.00% (16/32 people, 172 cases) in the control group. There was a statistically significant difference between the test groups and the control group (each p<0.0001). Systemic adverse drug reactions and other adverse drug reactions were not reported in Test Group 1 and the control group, and occurred in 6.45% (2/31 people, 2 cases) and 3.23% (1/31 people, 1 case) in Test Group 2, respectively. There was no statistically significant difference between Test Group 2 and the control group (systemic adverse drug reactions p=0.2381, other adverse drug reactions p=0.4921).

Serious adverse events, serious local adverse events, serious systemic adverse events, serious other adverse drug reactions, serious adverse drug reactions, serious local adverse drug reactions, serious systemic adverse drug reactions, and serious other adverse drug reactions were not reported in any group.

The occurrence status of adverse events is presented in Table 12, and the occurrence status of each local adverse drug reaction investigation item is presented in Table 13.

[Table 12]

| | AYP-101 2.5% (5 mg/cm$^2$) (n=33) n(%), [Number of occurrence] | AYP-101 5.0% (10 mg/cm$^2$) (n=31) n(%), [Number of occurrence] | Placebo of AYP-101 (0 mg/cm$^2$) (n=32) n(%), [Number of occurrence] |
|---|---|---|---|
| Adverse events | 33(100.00),[532] | 30(96.77),[538] | 20(62.50),[180] |
| 95% CI | [100.00,100.00] | [90.55,100.00] | [45.73,79.27] |
| Difference[95% CI] | 37.50[20.73,54.27] | 34.27[16.38,52.16] | |
| p-value[b] | <0.0001C | 0.0008C | |
| Local adverse events | 33(100.00),[525] | 30(96.77),[529] | 16(50.00),[172] |
| 95% CI | [100.00,100.00] | [90.55,100.00] | [32.68,67.32] |
| Difference[95% CI] | 50.00[32.68,67.32] | 46.77[28.37,65.18] | |
| p-value[b] | <0.0001C | <0.0001C | |
| Systemic adverse events | 1(3.03),[1] | 3(9.68),[3] | 2(6.25),[2] |
| 95% CI | [0.00,8.88] | [0.00,20.08] | [0.00,14.64] |
| Difference[95% CI] | -3.22[-13.44,7.01] | 3.43[-9.94,16.79] | |
| p-value[b] | 0.6132F | 0.6719F | |
| Other adverse events | 6(18.18),[6] | 5(16.13),[6] | 6(18.75),[6] |
| 95% CI | [5.02,31.34] | [3.18,29.08] | [5.23,32.27] |
| Difference[95% CI] | -0.57[-19.44,18.30] | -2.62[-21.34,16.10] | |
| p-value[b] | 0.9529C | 0.7841C | |
| Adverse drug reactions | 33(100.00),[525] | 30(96.77),[532] | 16(50.00),[172] |
| 95% CI | [100.00,100.00] | [90.55,100.00] | [32.68,67.32] |
| Difference[95% CI] | 50.00[32.68,67.32] | 46.77[28.37,65.18] | |
| p-value[b] | <0.0001C | <0.0001C | |
| Local adverse drug reactions | 33(100.00),[525] | 30(96.77),[529] | 16(50.00),[172] |
| 95% CI | [100.00,100.00] | [90.55,100.00] | [32.68,67.32] |

(continued)

| | AYP-101 2.5% (5 mg/cm$^2$) (n=33) n(%), [Number of occurrence] | AYP-101 5.0% (10 mg/cm$^2$) (n=31) n(%), [Number of occurrence] | Placebo of AYP-101 (0 mg/cm$^2$) (n=32) n(%), [Number of occurrence] |
|---|---|---|---|
| Difference[95% CI] | 50.00[32.68,67.32] | 46.77[28.37,65.18] | |
| p-value[b] | <0.0001C | <0.0001C | |
| Systemic adverse drug reactions | 0(0.00),[0] | 2(6.45),[2] | 0(0.00),[0] |
| 95% CI | [0.00,0.00] | [0.00,15.10] | [0.00,0.00] |
| Difference[95% CI] | NA | 6.45[-2.20,15.10] | |
| p-value[b] | NA | 0.2381F | |
| Other adverse drug reactions | 0(0.00),[0] | 1(3.23),[1] | 0(0.00),[0] |
| 95% CI | [0.00,0.00] | [0.00,9.45] | [0.00,0.00] |
| Difference[95% CI] | NA | 3.23[-2.99,9.45] | |
| p-value[b] | NA | 0.4921F | |
| Serious adverse events | 0(0.00),[0] | 0(0.00),[0] | 0(0.00),[0] |
| 95% CI | [0.00,0.00] | [0.00,0.00] | [0.00,0.00] |
| Difference[95% CI] | NA | NA | |
| p-value[b] | NA | NA | |
| Serious local adverse events | 0(0.00),[0] | 0(0.00),[0] | 0(0.00),[0] |
| 95% CI | [0.00,0.00] | [0.00,0.00] | [0.00,0.00] |
| Difference[95% CI] | NA | NA | |
| p-value[b] | NA | NA | |
| Serious systemic adverse events | 0(0.00),[0] | 0(0.00),[0] | 0(0.00),[0] |
| 95% CI | [0.00,0.00] | [0.00,0.00] | [0.00,0.00] |
| Difference[95% CI] | NA | NA | |
| p-value[b] | NA | NA | |
| Other serious adverse events | 0(0.00),[0] | 0(0.00),[0] | 0(0.00),[0] |
| 95% CI | [0.00,0.00] | [0.00,0.00] | [0.00,0.00] |
| Difference[95% CI] | NA | NA | |
| p-value[b] | NA | NA | |
| Serious adverse drug reactions | 0(0.00),[0] | 0(0.00),[0] | 0(0.00),[0] |
| 95% CI | [0.00,0.00] | [0.00,0.00] | [0.00,0.00] |
| Difference[95% CI] | NA | NA | |
| p-value[b] | NA | NA | |
| Serious local adverse drug reactions | 0(0.00),[0] | 0(0.00),[0] | 0(0.00),[0] |
| 95% CI | [0.00,0.00] | [0.00,0.00] | [0.00,0.00] |
| Difference[95% CI] | NA | NA | |
| p-value[b] | NA | NA | |
| Serious systemic adverse drug reactions | 0(0.00),[0] | 0(0.00),[0] | 0(0.00),[0] |
| 95% CI | [0.00,0.00] | [0.00,0.00] | [0.00,0.00] |

(continued)

| | AYP-101 2.5% (5 mg/cm$^2$) (n=33) n(%), [Number of occurrence] | AYP-101 5.0% (10 mg/cm$^2$) (n=31) n(%), [Number of occurrence] | Placebo of AYP-101 (0 mg/cm$^2$) (n=32) n(%), [Number of occurrence] |
|---|---|---|---|
| Difference[95% CI] | NA | NA | |
| p-value[b] | NA | NA | |
| Other serious adverse drug reactions | 0(0.00),[0] | 0(0.00),[0] | 0(0.00),[0] |
| 95% CI | [0.00,0.00] | [0.00,0.00] | [0.00,0.00] |
| Difference[95% CI] | NA | NA | |
| p-value[b] | NA | NA | |
| Numbers are 'number of subjects(%), [number of events]'<br>95% CI: 95% normal approximation confidence interval<br>C: Chi-square test/ F:Fisher's exact test/ NA: Not applicable<br>p-value[b]: Test between treatment groups | | | |

[Table 13]

| | AYP-101 2.5% (5 mg/$cm^2$) (n=33) n(%), [Number of occurrence] | AYP-101 5.0% (10 mg/$cm^2$) (n=31) n(%), [Number of occurrence] | Placebo of AYP-101 (0 mg/$cm^2$) (n=32) n(%), [Number of occurrence] |
|---|---|---|---|
| Local adverse drug reactions | 33(100.00), [525(98.68)] | 30(96.77), [529(98.33)] | 16(50.00), [172(95.56)] |
| 95% CI | [100.00,100.00] | [90.55,100.00] | [32.68,67.32] |
| Difference[95% CI] | 50.00[32.68,67.32] | 46.77[28.37,65.18] | |
| p-value[b] | <0.0001C | <0.0001C | |
| Severity | | | |
| Mean±Std | 1.01±0.09 | 1.04±0.15 | 1.01±0.07 |
| Median | 1.00 | 1.00 | 1.00 |
| Min, Max | 1.00, 2.00 | 1.00, 1.88 | 1.00, 1.50 |
| p-value[b] | 0.6795W | 0.0010W | |
| Maximum severity | | | |
| Mild | 33(100.00), [513(96.43)] | 30(96.77), [479(89.03)] | 16(50.00), [169(93.89)] |
| Moderate | 6(18.18),[11(2.07)] | 5(16.13),[50(9.29)] | 1(3.13),[3(1.67)] |
| Severe | 1(3.03),[1(0.19)] | 0(0.00),[0(0.00)] | 0(0.00),[0(0.00)] |
| Results | | | |
| Recovered | 33(100.00), [525(98.68)] | 30(96.77), [529(98.33)] | 16(50.00), [172(95.56)] |
| Recovering | 0(0.00),[0(0.00)] | 0(0.00),[0(0.00)] | 0(0.00),[0(0.00)] |
| Not recovered | 0(0.00),[0(0.00)] | 0(0.00),[0(0.00)] | 0(0.00),[0(0.00)] |
| Recovered but showing aftereffects | 0(0.00),[0(0.00)] | 0(0.00),[0(0.00)] | 0(0.00),[0(0.00)] |
| Dead due to ad-verse drug reac-tions | 0(0.00),[0(0.00)] | 0(0.00),[0(0.00)] | 0(0.00),[0(0.00)] |
| Unknown | 0(0.00),[0(0.00)] | 0(0.00),[0(0.00)] | 0(0.00),[0(0.00)] |
| Causal relationship with drugs for clinical trials | | | |
| Relevant - certain | 33(100.00), [525(98.68)] | 30(96.77), [529(98.33)] | 16(50.00), [172(95.56)] |

(continued)

| | | | |
|---|---|---|---|
| Causal relationship with drugs for clinical trials | | | |
| Relevant - fairly certain | 0(0.00),[0(0.00)] | 0(0.00),[0(0.00)] | 0(0.00),[0(0.00)] |
| Relevant - possible | 0(0.00),[0(0.00)] | 0(0.00),[0(0.00)] | 0(0.00),[0(0.00)] |
| Relevant - difficult to evaluate | 0(0.00),[0(0.00)] | 0(0.00),[0(0.00)] | 0(0.00),[0(0.00)] |
| Relevant - cannot be evaluated | 0(0.00),[0(0.00)] | 0(0.00),[0(0.00)] | 0(0.00),[0(0.00)] |
| Not relevant - less likely | 0(0.00),[0(0.00)] | 0(0.00),[0(0.00)] | 0(0.00),[0(0.00)] |
| Measures for drugs for clinical trials | | | |
| Administration stopped | 0(0.00),[0(0.00)] | 0(0.00),[0(0.00)] | 0(0.00),[0(0.00)] |
| Dosage decreased | 0(0.00),[0(0.00)] | 0(0.00),[0(0.00)] | 0(0.00),[0(0.00)] |
| Dosage increased | 0(0.00),[0(0.00)] | 0(0.00),[0(0.00)] | 0(0.00),[0(0.00)] |
| Dosage unchanged | 33(100.00), [525(98.68)] | 30(96.77), [529(98.33)] | 16(50.00), [172(95.56)] |
| Uncertain | 0(0.00),[0(0.00)] | 0(0.00),[0(0.00)] | 0(0.00),[0(0.00)] |
| None | 0(0.00),[0(0.00)] | 0(0.00),[0(0.00)] | 0(0.00),[0(0.00)] |
| Measures other than drugs for clinical trials | | | |
| No action | 33(100.00), [525(98.68)] | 30(96.77), [529(98.33)] | 16(50.00), [172(95.56)] |
| Drug treatment | 0(0.00),[0(0.00)] | 0(0.00),[0(0.00)] | 0(0.00),[0(0.00)] |
| Non-drug treatment | 0(0.00),[0(0.00)] | 0(0.00),[0(0.00)] | 0(0.00),[0(0.00)] |
| Drug/Non-drug treatment | 0(0.00),[0(0.00)] | 0(0.00),[0(0.00)] | 0(0.00),[0(0.00)] |
| Duration | | | |
| 1-3 days | 31(93.94), [384(72.18)] | 27(87.10), [316(58.74)] | 16(50.00), [141(78.33)] |
| 4-7 days | 28(84.85), [127(23.87)] | 24(77.42), [186(34.57)] | 6(18.75), [24(13.33)] |

(continued)

| Duration | | | |
|---|---|---|---|
| 8-14 days | 7(21.21),[14(2.63)] | 8(25.81),[26(4.83)] | 4(12.50),[6(3.33)] |
| 15-30 days | 0(0.00),[0(0.00)] | 1(3.23),[1(0.19)] | 0(0.00),[0(0.00)] |
| 31-60 days | 0(0.00),[0(0.00)] | 0(0.00),[0(0.00)] | 1(3.13),[1(0.56)] |
| >60 days | 0(0.00),[0(0.00)] | 0(0.00),[0(0.00)] | 0(0.00),[0(0.00)] |
| Ongoing | 0(0.00),[0(0.00)] | 0(0.00),[0(0.00)] | 0(0.00),[0(0.00)] |

Numbers are 'number of subjects(%),[number of events(%)]'
[number of events (%)]: (number of local adverse drug reactions for the relevant item / total number of adverse drug reactions for the relevant item) * 10095% CI: 95% normal approximation confidence interval
C: Chi-square test/ F: Fisher's exact test/ T: Two sample t-test/ W: Wilcoxon's rank sum test/ NA: Not applicable
p-value[b]: Test between treatment groups
Note. Severity: mild (1), moderate (2), severe (3)

$$\text{Duration (days)} = \text{Day of elimination of adverse event} - \text{Date of occurrence} + 1$$

The injectable composition for localized fat reduction of the present invention, comprising taurocholic acid or glycocholic acid (or a salt thereof) and phosphatidylcholine (PPC) in a specific mixing ratio, provides a safe and stable formulation and is highly effective in reducing fat cells without various side effects, and may be administered to the affected area at shorter intervals to maximize the effects.

## Claims

1. A composition for localized fat reduction with reduced pain and side effects, comprising:

   (i) phosphatidyl choline; and
   (ii) at least one selected from the group consisting of glycocholic acid (GCA), taurocholic acid (TCA) and salts thereof,
   wherein (i) and (ii) have a molar ratio of (ii)/(i) of 0.7 to 3.0, and
   wherein the composition is administered to the affected area of a subject at intervals of less than 4 weeks at a concentration of 25 to 50 mg/mL.

2. The composition for localized fat reduction of claim 1,
   wherein the composition is administered to the affected area of a subject at intervals of 3 weeks or less.

3. The composition for localized fat reduction of claim 1,
   wherein the composition is administered to the affected area of a subject at intervals of 2 weeks or less.

4. The composition for localized fat reduction of claim 1,
   wherein the composition is administered to the affected area of subject a total of 2 to 10 times.

5. The composition for localized fat reduction of claim 1,
   wherein the composition is administered to 1 to 50 target sites at grid intervals of 0.5 to 2.0 cm during one administration.

6. The composition for localized fat reduction of claim 5,
   wherein the composition is administered in an amount of 0.1 to 0.5 mL per target site.

7. The composition for localized fat reduction of claim 5,
   wherein the composition is administered 2.5 mg to 25 mg of phosphatidylcholine per target site.

8. The composition for localized fat reduction of claim 5,
   where the composition is administered 5 mg to 10 mg of phosphatidylcholine per target site.

9. The composition for localized fat reduction of claim 1,
   wherein the composition is administered to one or more areas selected from the group consisting of eyelids, under the eyes, under the chin, back, arms, legs, brassiere line, flanks, abdomen, buttocks, thighs, and calves.

[FIG. 1]

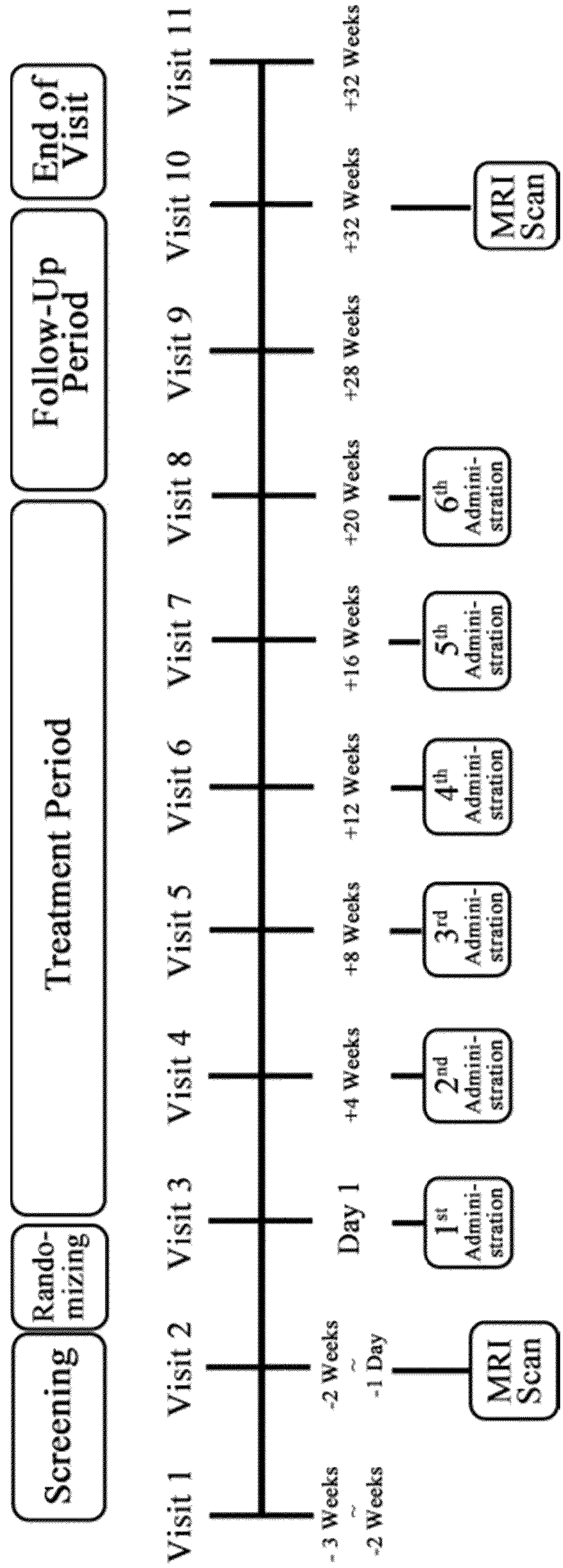
Screening
Rando-
mizing
Treatment Period
Follow-Up
Period
End of
Visit
Visit 1
Visit 2
Visit 3
Visit 4
Visit 5
Visit 6
Visit 7
Visit 8
Visit 9
Visit 10
Visit 11
- 3 Weeks
~
-2 Weeks
-2 Weeks
~
-1 Day
Day 1
+4 Weeks
+8 Weeks
+12 Weeks
+16 Weeks
+20 Weeks
+28 Weeks
+32 Weeks
+32 Weeks
MRI
Scan
1st
Admini-
stration
2nd
Admini-
stration
3rd
Admini-
stration
4th
Admini-
stration
5th
Admini-
stration
6th
Admini-
stration
MRI
Scan

[FIG. 2]

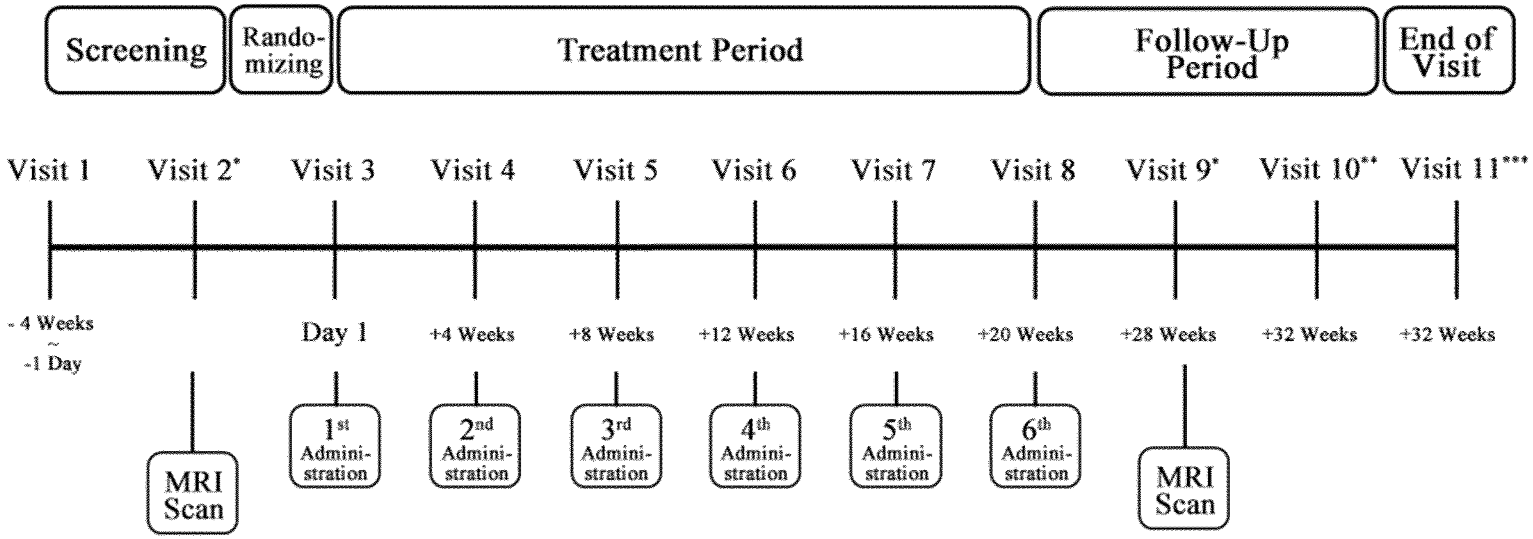


* The suitability for MRI imaging was confirmed on Visit 1, and MRI imaging was performed on selected subjects on Visits 2 and 9.
(MRI imaging was performed with the same device designated by the institution, and imaging could be performed on Visit 1 and Visit 10 depending on the reservation status of the MRI imaging.)

** The time point of analysis of the main endpoint: 4 weeks after the final administration of drugs for clinical trials.

*** The time point of the end point of the trial: 12 weeks after the final administration of drugs for clinical trials.

[FIG. 3]

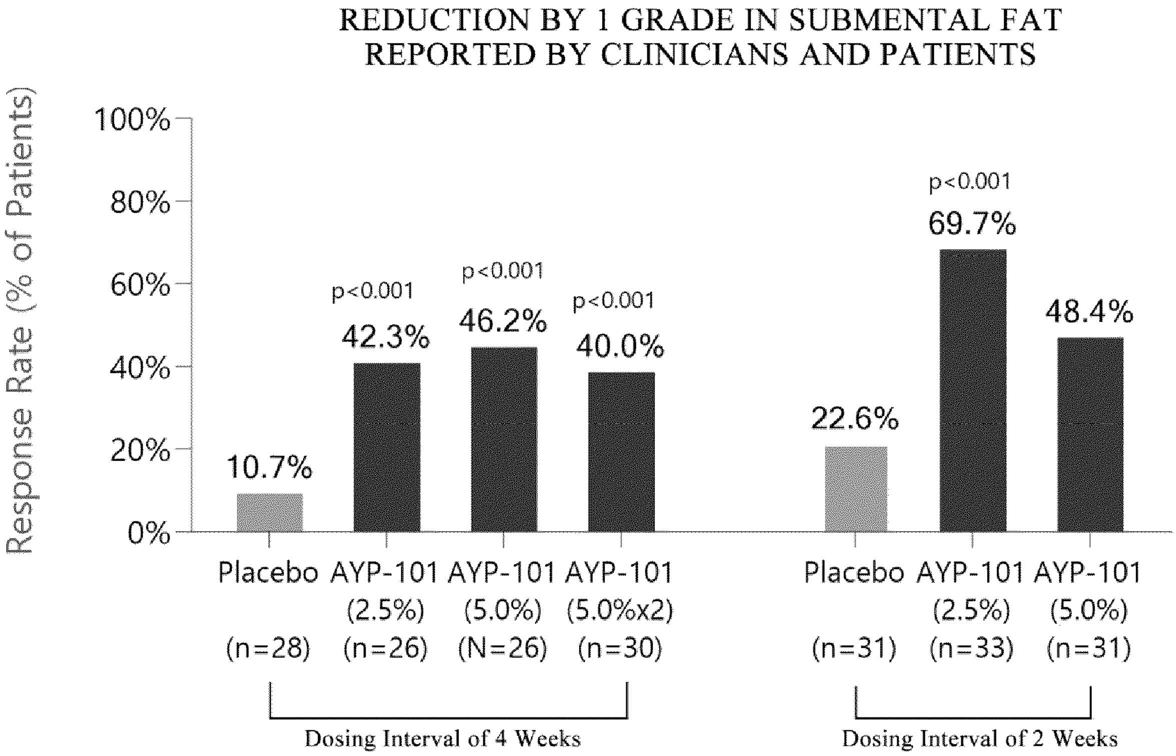
REDUCTION BY 1 GRADE IN SUBMENTAL FAT
REPORTED BY CLINICIANS AND PATIENTS
Response Rate (% of Patients)
100%
80%
60%
40%
20%
0%
p<0.001
p<0.001
p<0.001
p<0.001
10.7%
42.3%
46.2%
40.0%
22.6%
69.7%
48.4%
Placebo (n=28)
AYP-101 (2.5%) (n=26)
AYP-101 (5.0%) (N=26)
AYP-101 (5.0%x2) (n=30)
Placebo (n=31)
AYP-101 (2.5%) (n=33)
AYP-101 (5.0%) (n=31)
Dosing Interval of 4 Weeks
Dosing Interval of 2 Weeks

Europäisches Patentamt
European Patent Office
Office européen des brevets

# EUROPEAN SEARCH REPORT

Application Number
EP 24 20 5448

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 3 479 827 B1 (AMI PHARM CO LTD [KR]) 10 May 2023 (2023-05-10)<br>* claims 1-8 *<br>* paragraphs [0001], [0017] - [0026], [0035] - [0043], [0148] - [0151], [0175], [0181], [0190] *<br>* table 14 *<br>* example 6 *<br>----- | 1-9 | INV.<br>A61K8/55<br>A61K8/63<br>A61K9/00<br>A61K31/575<br>A61K31/685<br>A61P43/00<br>A61Q19/06 |
| | | | TECHNICAL FIELDS SEARCHED (IPC) |
| | | | A61K<br>A61Q<br>A61P |

The present search report has been drawn up for all claims

1

| Place of search | Date of completion of the search | Examiner |
|---|---|---|
| The Hague | 14 March 2025 | De Masi, Alessia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 20 5448

This annex lists the patent family members relating to the patent documents cited in the above-mentioned European search report. The members are as contained in the European Patent Office EDP file on
The European Patent Office is in no way liable for these particulars which are merely given for the purpose of information.

14-03-2025

| Patent document cited in search report | Publication date | Patent family member(s) | Publication date |
|---|---|---|---|
| EP 3479827 B1 | 10-05-2023 | AU 2018256263 A1 | 16-05-2019 |
| | | BR 112019008065 A2 | 02-07-2019 |
| | | CA 3031678 A1 | 25-10-2018 |
| | | CN 109862897 A | 07-06-2019 |
| | | CO 2019004317 A2 | 10-05-2019 |
| | | EP 3479827 A1 | 08-05-2019 |
| | | ES 2949946 T3 | 04-10-2023 |
| | | IL 266873 A | 29-08-2019 |
| | | JP 7034497 B2 | 14-03-2022 |
| | | JP 2020517581 A | 18-06-2020 |
| | | KR 101887586 B1 | 10-08-2018 |
| | | RU 2736974 C1 | 23-11-2020 |
| | | US 2019201328 A1 | 04-07-2019 |
| | | WO 2018194427 A1 | 25-10-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1887586


### Non-patent literature cited in the description


- **A.GUPTA**. Action and comparative efficacy of phosphatidylcholine formulation and isolated sodium deoxycholate for different cell type. *Aest Plast Sur*, 2009, vol. 33, 346-352
- **DONG-SEOK KIM**. Phosphatidylcholine induces apoptosis of 3T3-L1 adipocytes. *Journal of biomedical science*, 2011, vol. 18 (91), 1-7
- **SHAMBAN AT**. Noninvasive Submental Fat Compartment Treatment. *Plast Reconstr Surg Glob Open*, 14 December 2016, vol. 4 (12), e1155
- **THOMAS MK** ; **D'SILVA JA** ; **BOROLE AJ**. Injection Lipolysis: A Systematic Review of Literature and Our Experience with a Combination of Phosphatidylcholine and Deoxycholate over a Period of 14 Years in 1269 Patients of Indian and South East Asian Origin. *J Cutan Aesthet Surg.*, October 2018, vol. 11 (4), 222-228
- KYBELLA (deoxycholic acid) injection. *Allergan*, 2018
- **ADAM M. ROTUNDA** ; **STEVEN R. WEISS** ; **LARRY S. RIVKIN**. Randomized Double-Blind Clinical Trial of Subcutaneously Injected Deoxycholate Versus a Phosphatidylcholine-Deoxycholate Combination for the Reduction of Submental Fat. *Dermatol Surg*, 2009, vol. 35, 792-803